# EUROPEAN PATENT APPLICATION

(11) **EP 4 670 619 A1**
(43) Date of publication of application: **31.12.2025**
(21) Application number: 23930840.6
(22) Date of filing: 17.11.2023
(51) Int. Cl.: A61B 3/15

(54) **OPHTHALMOLOGIC DEVICE AND METHOD FOR OPERATING OPHTHALMOLOGIC DEVICE**

(30) Priority: 28.03.2023 JP 2023052058
(71) Applicant: Topcon Corporation, Tokyo 174-8580 (JP)
(72) Inventor: SHIBANO, Takaaki, Tokyo 174-8580 (JP); MOCHIZUKI, Yuki, Tokyo 174-8580 (JP); SUZUKI, Minami, Tokyo 174-8580 (JP); YAMABE, Masaru, Tokyo 174-8580 (JP); TSUKIHARA, Kouichi, Tokyo 174-8580 (JP)
(74) Representative: Schmitt-Nilson Schraud Waibel Wohlfrom Patentanwälte Partnerschaft mbB
(86) International application number: PCT/JP2023/041453
(87) International publication number: WO 2024/202213

(57) **Abstract**

There are provided an ophthalmic device capable of reliably avoiding an examination head coming close to a nose of a subject and a method for operating the ophthalmic device. The ophthalmic device includes a displacement mechanism (an XZ movement mechanism (16), a Y movement mechanism (18), a swing rotation mechanism (20), and a tilt rotation mechanism (80)) configured to displace an examination head (22) with respect to subject eyes (E), a drive controlling unit (46) configured to drive the displacement mechanism to displace the examination head (22) to an examination position for the subject eye (E) under examination along an axis (TA) of tilt, wherein the axis (TA) of tilt is an axis obtained by tilting, around a center of the subject eye (E) under examination, a reference axis (VA) that extends along an eye direction of the subject eye (E) in parallel with a front-back direction (Z-direction) serving as an operating distance direction of the examination head (22), outward away from a subject's nose (N), and a retraction controlling unit (54) configured to, when examination on a first eye of the subject eyes (E) by the examination head (22) is completed, drive the displacement mechanism to retract the examination head (22) in a direction away from a face of the subject.

## Description

### {Technical Field}

The presently disclosed subject matter relates to an ophthalmic device which sequentially examines left and right subject eyes using an examination head and a method for operating the ophthalmic device.

### {Background Art}

In ophthalmology, ophthalmic examinations (e.g., acquisition of various ocular characteristics, such as ocular refractive power, an intraocular pressure, and the number of corneal endothelial cells, of a subject eye, fundus imaging, and tomography) on the subject eye are performed by an ophthalmic device. Alignment of an examination head (also referred to as an examination unit) of the ophthalmic device with respect to the subject eye is extremely important in terms of, e.g., accuracy, reliability, and image quality of a result of the examinations on the subject eye.

For this reason, each of the ophthalmic devices according to Patent Literatures 1 and 2 stereoscopically photographs a subject eye using a stereo camera and executes alignment detection that detects a relative position of the subject eye to an examination head based on anterior eye part images of the subject eye obtained by the stereoscopic photographing. The ophthalmic device moves the examination head through electromotive driving based on a result of the alignment detection, thereby executing automatic alignment of the examination head with the subject eye. After completion of the automatic alignment, examination (e.g., photography) on the subject eye is executed by the examination head.

In examining left and right subject eyes by the examination head, after completion of examination on one (a first eye) of the left and right subject eyes by the examination head, alignment of the examination head with the other (a second eye) of the left and right subject eyes (left-right eye switching) and examination on the second eye by the examination head are executed.

### {Citation List}

### {Patent Literature}

{Patent Literature 1} Japanese Patent Application Laid-Open No. 2013-248376
{Patent Literature 2} Japanese Patent Application Laid-Open No. 2021-069415

### {Summary of Invention}

### {Technical Problem}

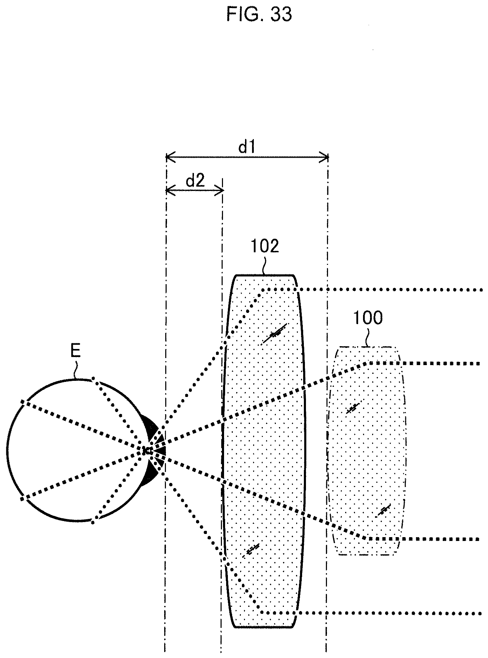

Figure 33 is an explanatory diagram for explaining a relationship between a lens size of an objective lens in an examination head and an operating distance of the examination head. As illustrated in Figure 33, a photographic angle of view of a conventional objective lens 100 in an examination head is about 45°. An ophthalmic device such as a fundus camera, an Optical Coherence Tomography (OCT) device, or a Scanning Laser Ophthalmoscope (SLO) device supporting wide-angle photographing has an objective lens 102 larger than the objective lens 100 in an examination head due to optical constraints. As a result, an operating distance d2 between a subject eye E and the examination head with the objective lens 102 is shorter than an operating distance d1 with the objective lens 100.

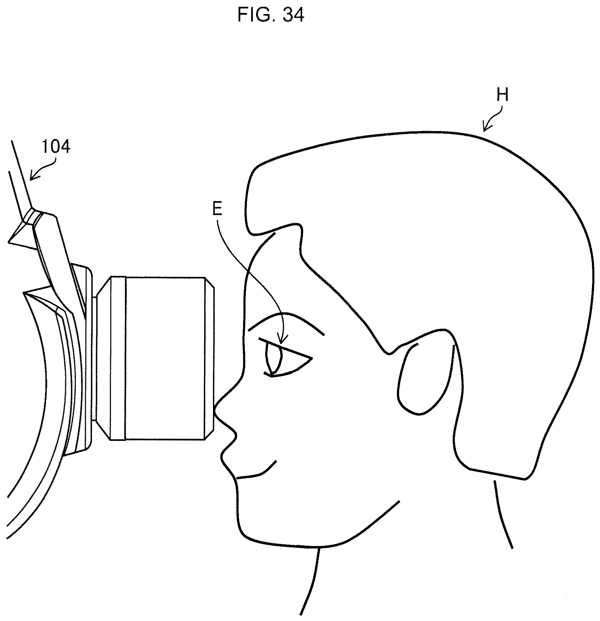

Figure 34 is an explanatory view for explaining a problem arising from a short operating distance between the subject eye E and an examination head 104. For example, when an objective lens diameter of the examination head 104 is 80 mm, and an operating distance of the examination head 104 is 50 mm, as illustrated in Figure 34, a distance between a forehead of the subject H and the examination head 104 is about 22 mm, a distance between a nose of the subject H and the examination head 104 is about 2 mm, and a distance between cheeks of the subject H and the examination head 104 is about 28 mm. The distance between the nose of the subject H and the examination head 104 is thus particularly short. For this reason, when the subject H moves his/her face during left-right eye switching after completion of examination on the subject eye E (a first eye), the examination head 104 may come closer to the face (the nose to be specific) of the subject H).

The presently disclosed subject matter has been made in view of the above-described circumstances, and has as its object to provide an ophthalmic device capable of reliably avoiding an examination head coming closer to a nose of a subject and a method for operating the ophthalmic device.

### {Solution to Problem}

An ophthalmic device for achieving the object of the presently disclosed subject matter is an ophthalmic device including: an examination head configured to examine subject eyes, wherein the ophthalmic device is configured to use the examination head to examine a first eye that is one of left and right ones of the subject eyes and then examine a second eye that is the other of the left and right subject eyes; a displacement mechanism configured to displace the examination head with respect to the subject eyes; a drive controlling unit configured to drive the displacement mechanism to displace the examination head to an examination position for the subject eye under examination along an axis of tilt, wherein the axis of tilt is an axis obtained by tilting, around a center of one subject eye under examination, a reference axis that extends along an eye direction of the subject eye in parallel with a front-back direction serving as an operating distance direction of the examination head, outward away from a subject's nose; and a retraction controlling unit configured to, when examination on the first eye by the examination head is completed, drive the displacement mechanism to retract the examination head in a direction away from a face of the subject.

According to the ophthalmic device, it is possible to avoid the examination head coming close to the face of the subject even if motion of the face of the subject occurs after completion of the examination on the first eye.

In the ophthalmic device according to another aspect of the presently disclosed subject matter, the retraction controlling unit drives the displacement mechanism to retract the examination head in a direction away from the nose in the face. This makes it possible to avoid the examination head coming closer to the subject's nose after completion of the examination on the first eye.

In the ophthalmic device according to another aspect of the presently disclosed subject matter, the retraction controlling unit drives the displacement mechanism to retract the examination head in a direction away from the face along the axis of tilt. This makes it possible to avoid the examination head coming closer to the face of the subject after completion of the examination on the first eye.

In the ophthalmic device according to another aspect of the presently disclosed subject matter, the displacement mechanism includes a movement mechanism configured to move the examination head in the front-back direction, a left-right direction, and an up-down direction with respect to the subject eyes, and a rotation mechanism configured to rotate the examination head around a rotation axis determined in advance, and when a direction, in which the examination head moves away from the face, of the front-back direction, is a rearward direction, the retraction controlling unit drives the movement mechanism to move the examination head in at least one of the rearward direction and the outward direction. This makes it possible to avoid the examination head coming closer to the face of the subject after completion of the examination on the first eye.

In the ophthalmic device according to another aspect of the presently disclosed subject matter, the displacement mechanism includes a movement mechanism configured to move the examination head in the front-back direction, a left-right direction, and an up-down direction with respect to the subject eyes, and a rotation mechanism configured to rotate the examination head around a rotation axis determined in advance, and the retraction controlling unit drives the rotation mechanism to rotate the examination head in a direction away from the nose. This makes it possible to avoid the examination head coming close to the subject's nose after completion of the examination on the first eye.

In the ophthalmic device according to another aspect of the presently disclosed subject matter, the retraction controlling unit drives the displacement mechanism to retract the examination head in an upward direction. This makes it possible to avoid the examination head coming close to the face of the subject after completion of the examination on the first eye.

The ophthalmic device according to another aspect of the presently disclosed subject matter includes a photographing unit configured to photograph the subject's nose, and a nose position detecting unit configured to detect a position of the nose based on a photographed image of the nose photographed by the photographing unit, and the retraction controlling unit drives the displacement mechanism based on a result of detection from the nose position detecting unit to retract the examination head in a direction away from the face of the subject. This makes it possible to reliably retract the examination head in the direction away from the face of the subject.

The ophthalmic device according to another aspect of the presently disclosed subject matter includes a motion detecting unit configured to continuously detect presence or absence of motion of the face while the retraction controlling unit drives the displacement mechanism to retract the examination head in a direction away from the face, and the retraction controlling unit stops driving of the displacement mechanism or increases a velocity of retraction of the examination head by the displacement mechanism when motion of the face is detected by the motion detecting unit. This makes it possible to prevent the examination head from coming closer to the face (the nose to be specific) of the subject even if motion of the face of the subject occurs during retraction of the examination head.

In the ophthalmic device according to another aspect of the presently disclosed subject matter, the rotation axis is parallel to the up-down direction, and the outward direction is parallel to the left-right direction.

In the ophthalmic device according to another aspect of the presently disclosed subject matter, the rotation axis is perpendicular to the up-down direction, and the outward direction is an upward direction of the up-down direction.

In the ophthalmic device according to another aspect of the presently disclosed subject matter, the displacement mechanism includes a movement mechanism configured to move the examination head in the front-back direction, a left-right direction, and an up-down direction with respect to the subject eyes, and a rotation mechanism configured to rotate the examination head around a rotation axis determined in advance, the examination head includes an objective lens, the drive controlling unit executes a process of driving the movement mechanism to move the examination head from respective initial positions determined in advance for the left and right subject eyes to the axis of tilt, a process of driving the rotation mechanism to rotate the examination head and make an optical axis of the objective lens parallel to the axis of tilt, and a process of driving the movement mechanism to displace the examination head to the examination position along the axis of tilt, and the device includes a left-right eye switching controlling unit configured to, after the retraction controlling unit drives the displacement mechanism to retract the examination head in a direction away from the face, drive the displacement mechanism to displace the examination head to a second initial position that is the initial position for the second eye via a first initial position that is the initial position for the first eye or to displace the examination head to the second initial position not via the first initial position.

In the ophthalmic device according to another aspect of the presently disclosed subject matter, the rotation mechanism has the rotation axis parallel to the up-down direction and rotates the examination head around the objective lens, the first initial position and the second initial position are spaced in the left-right direction when viewed from a one-direction side in the up-down direction, the left-right eye switching controlling unit executes, in displacing the examination head from the first initial position to the second initial position, a left-right movement process of driving the movement mechanism to move the examination head at least in the left-right direction and a rotation process of making the optical axis parallel to the reference axes by driving the rotation mechanism to rotate the examination head during the left-right movement process, and the device includes a photographing unit provided at the examination head and configured to photograph the subject's nose after execution of the rotation process, a nose position detecting unit configured to detect a position of the nose based on a photographed image of the nose photographed by the photographing unit, and a tilting angle determining unit configured to determine a tilting angle of the axis of tilt with respect to the reference axis corresponding to the second eye based on a result of detection from the nose position detecting unit. This makes it possible to determine a tilting angle corresponding to a position of the face of the subject before examination on the second eye. Thus, the examination head is prevented from coming closer to the face (the nose to be specific) of the subject in displacing the examination head to an examination position for the second eye.

A method for operating an ophthalmic device for achieving the object of the presently disclosed subject matter is a method for operating an ophthalmic device including an examination head configured to examine subject eyes and a displacement mechanism configured to displace the examination head with respect to the subject eyes, wherein the ophthalmic device is configured to use the examination head to examine a first eye that is one of left and right ones of the subject eyes and then examine a second eye that is the other of the left and right subject eyes, the method including: a drive controlling step of driving the displacement mechanism to displace the examination head to an examination position for the subject eye under examination along an axis of tilt, wherein the axis of tilt is an axis obtained by tilting, around a center of one subject eye under examination, a reference axis that extends along an eye direction of the subject eye in parallel with a front-back direction serving as an operating distance direction of the examination head, outward away from a subject's nose; and a retraction controlling step of, when examination on the first eye by the examination head is completed, driving the displacement mechanism to retract the examination head in a direction away from a face of the subject.

### {Advantageous Effects of Invention}

According to the presently disclosed subject matter, it is possible to reliably avoid an examination head coming close to a subject's nose.

### {Brief Description of Drawings}

Figure 1 is a side view of an ophthalmic device according to a first embodiment;
Figure 2 is a front view of a lens-barrel as viewed from a front side in a Z direction;
Figure 3 is a cross-sectional view of the lens-barrel taken along line 3-3 in Figure 2;
Figure 4 is a block diagram illustrating a configuration of the ophthalmic device according to the first embodiment;
Figure 5 is an explanatory diagram for explaining a method for automatic alignment of an examination head;
Figure 6 is an explanatory diagram for explaining an example 1-1 of the automatic alignment of the examination head according to the first embodiment;
Figure 7 is an explanatory diagram for explaining an example 1-2 of the automatic alignment of the examination head according to the first embodiment;
Figure 8 is an explanatory diagram for explaining an example 1-3 of the automatic alignment of the examination head according to the first embodiment;
Figure 9 is an explanatory diagram for explaining an example 1-4 of the automatic alignment of the examination head according to the first embodiment;
Figure 10 is an explanatory diagram for explaining an example 1 of retraction control of the examination head;
Figure 11 is an explanatory diagram for explaining an example 2 of the retraction control of the examination head;
Figure 12 is an explanatory diagram for explaining an example 3 of the retraction control of the examination head;
Figure 13 is an explanatory diagram for explaining an example 4 of the retraction control of the examination head;
Figure 14 is an explanatory diagram for explaining an example 5 of the retraction control of the examination head;
Figure 15 is an explanatory diagram for explaining an example of a left-right eye switching controlling process to be executed by a left-right eye switching controlling unit;
Figure 16 is a flowchart illustrating a flow of a process of examining a subject eye by the ophthalmic device according to the first embodiment;
Figure 17 is a flowchart illustrating a flow of an automatic alignment process for the examination head;
Figure 18 is an explanatory diagram for explaining displacement of the examination head after the start of the automatic alignment;
Figure 19 is a side view of an ophthalmic device according to a second embodiment;
Figure 20 is an explanatory diagram for explaining an example 2-1 of automatic alignment of an examination head according to the second embodiment;
Figure 21 is an explanatory diagram for explaining an example 2-2 of the automatic alignment of the examination head according to the second embodiment;
Figure 22 is an explanatory diagram for explaining retraction control to be performed by a retraction controlling unit according to the second embodiment through driving of a rotation mechanism;
Figure 23 is a block diagram illustrating a configuration of an ophthalmic device according to a third embodiment;
Figure 24 is a flowchart illustrating a flow of retraction control of an examination head by the ophthalmic device according to the third embodiment;
Figure 25 is an explanatory view for explaining a modification of a method for detecting presence or absence of motion of a face during retraction of the examination head in the ophthalmic device according to the third embodiment;
Figure 26 is a block diagram illustrating a configuration of an ophthalmic device according to a fourth embodiment;
Figure 27 is an explanatory diagram for explaining an example of left-right eye switching control to be executed by a left-right eye switching controlling unit according to the fourth embodiment;
Figure 28 is a flowchart illustrating a flow of a left-right eye switching controlling process by the ophthalmic device according to the fourth embodiment;
Figure 29 is a side view of an ophthalmic device according to a fifth embodiment;
Figure 30 is an explanatory diagram for explaining an example 3 of automatic alignment of an examination head according to the fifth embodiment;
Figure 31 is a side view of an ophthalmic device according to a sixth embodiment;
Figure 32 is an explanatory diagram for explaining an example 4 of automatic alignment of an examination head according to the sixth embodiment;
Figure 33 is an explanatory diagram for explaining a relationship between a lens size of an objective lens in an examination head and an operating distance of the examination head; and
Figure 34 is an explanatory view for explaining a problem arising from a short operating distance between a subject eye and an examination head.

### {Description of Embodiments}

### [First Embodiment]

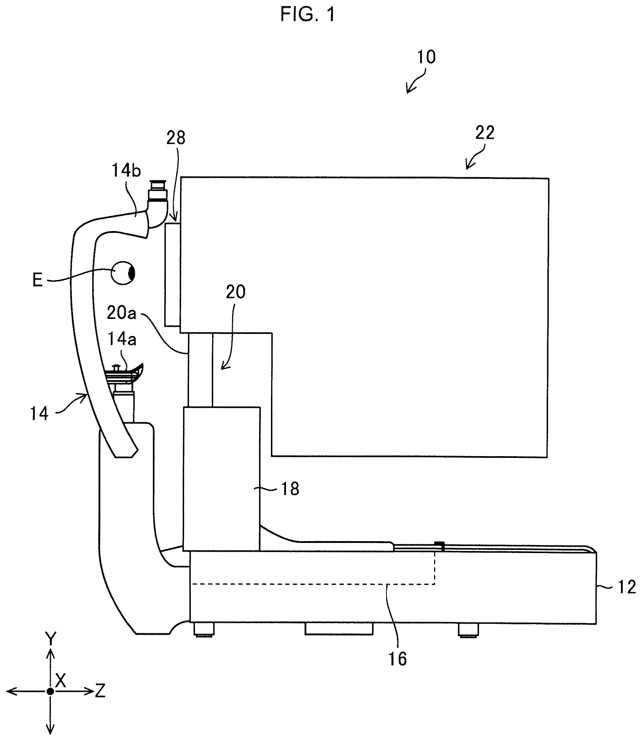

Figure 1 is a side view of an ophthalmic device 10 according to a first embodiment. An X direction in Figure 1 is a left-right direction based on a subject, a Y direction is an up-down direction, and that a Z direction is a front-back direction (also referred to as an operating distance direction) parallel to a forward direction toward the subject (a subject eye E) and a rearward direction away from the subject.

As illustrated in Figure 1, the ophthalmic device 10 is a multifunction machine which is a combination of a fundus camera that executes fundus imaging of the subject eye E and an optical coherence tomograph that obtains a tomographic image of the subject eye E using OCT. The ophthalmic device 10 includes a base 12, a face support 14, an XZ movement mechanism 16, a Y movement mechanism 18, a swing rotation mechanism 20, and an examination head 22.

The face support 14 is attached to a front end portion on a front side (a subject eye E side) in the Z direction of the base 12. The XZ movement mechanism 16 is also provided at the base 12.

The face support 14 includes a chin rest 14a and a forehead rest 14b which are positionally adjustable in the Y direction (up-down direction), and supports a face of the subject at a position facing the examination head 22 (a lens-barrel 28).

The XZ movement mechanism 16 together with the Y movement mechanism 18 (to be described later) constitutes a movement mechanism according to the presently disclosed subject matter. The XZ movement mechanism 16 includes a pedestal which is movable in each of the X and Z directions with respect to the base 12 and an electric drive mechanism (a publicly known actuator, such as a motor drive mechanism) which moves the pedestal in each of the X and Z directions (both not illustrated). The XZ movement mechanism 16 integrally moves the Y movement mechanism 18, the swing rotation mechanism 20, and the examination head 22 in the X and Z directions.

The Y movement mechanism 18 includes a lifting table which is movable in the Y direction and an electric drive mechanism which moves the lifting table in the Y direction (both not illustrated). The Y movement mechanism 18 integrally moves the swing rotation mechanism 20 and the examination head 22 in the Y direction. With this configuration, the XZ movement mechanism 16 and the Y movement mechanism 18 can integrally move the swing rotation mechanism 20 and the examination head 22 in the X, Y, and Z directions.

The swing rotation mechanism 20 corresponds to a rotation mechanism according to the presently disclosed subject matter and, together with the XZ movement mechanism 16 and the Y movement mechanism 18 described earlier, constitutes a displacement mechanism according to the presently disclosed subject matter. The swing rotation mechanism 20 includes a rotating shaft 20a parallel to the Y direction and an electric drive mechanism which rotates the rotating shaft 20a, and rotates (swings) the examination head 22 around the rotating shaft 20a.

The examination head 22 is attached to an upper end portion in the Y direction of the rotating shaft 20a. With this configuration, the examination head 22 is movable in the X, Y, and Z direction by the XZ movement mechanism 16 and the Y movement mechanism 18, and is rotatable in a direction around the rotating shaft 20a by the swing rotation mechanism 20.

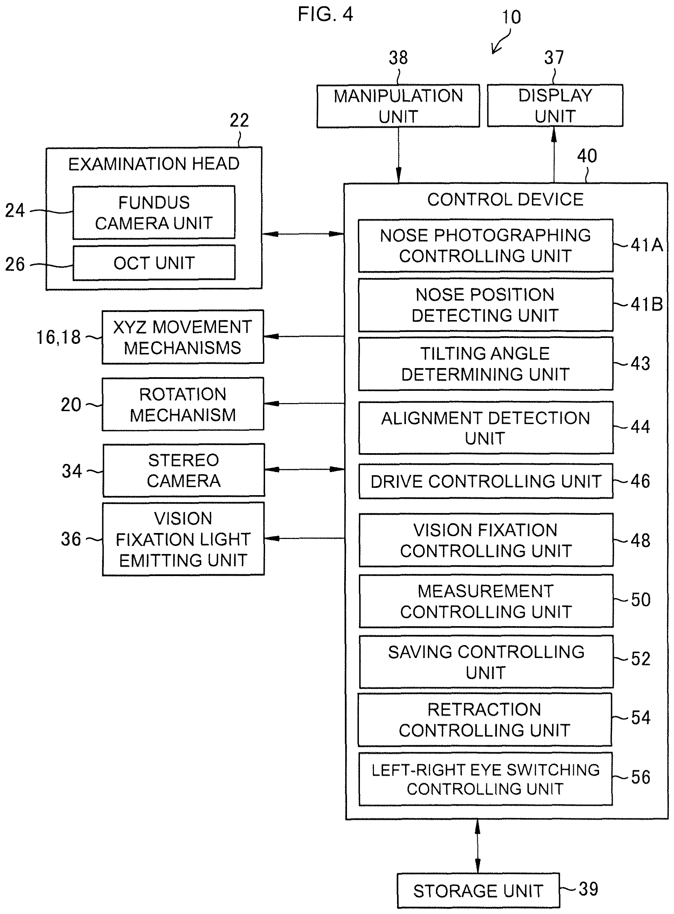

The examination head 22 includes a fundus camera unit 24 and an OCT unit 26 (to be described later) illustrated in Figure 4, and the lens-barrel 28. The fundus camera unit 24 photographs a fundus of the subject eye E through an objective lens 30 (to be described later) and outputs a fundus image which is a frontal image of the fundus to a control device 40 (to be described later) (see Figure 4). The OCT unit 26 performs OCT imaging of the subject eye E through the objective lens 30 and outputs a signal such as a detection signal needed to generate a tomographic image of the subject eye E to the control device 40. Specific configurations of the fundus camera unit 24 and the OCT unit 26 are publicly known techniques (see Patent Literature 1 described above) and that a specific description thereof will be omitted.

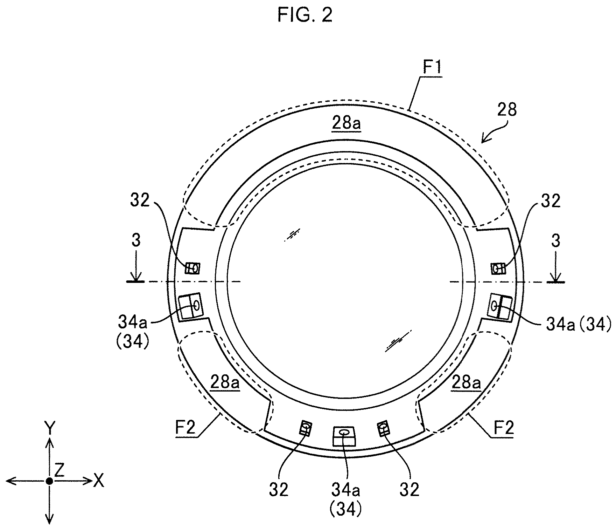

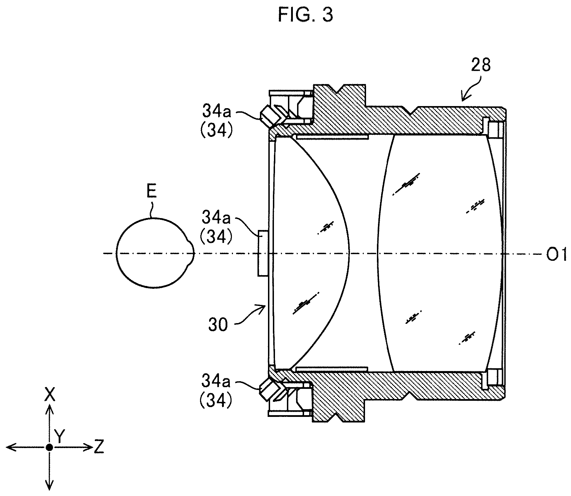

Figure 2 is a front view of the lens-barrel 28 as viewed from the front side in the Z direction. Figure 3 is a cross-sectional view of the lens-barrel 28 taken along line 3-3 in Figure 2. Figures 1 to 3 illustrate an example which provides the lens-barrel 28 at an end portion on the front side in the Z direction of the examination head 22. The lens-barrel 28 houses (holds) the objective lens 30 having an optical axis O1 (see Figure 3) parallel to the Z direction. Four illumination light sources 32 and a stereo camera 34 are provided at a lens-barrel distal end face 28a on the front side in the Z direction of the lens-barrel 28. As the objective lens 30, for example, a large lens supporting wide-angle photographing, i.e., a lens with a short operating distance is used (see Figure 33). The type of the objective lens 30 is not particularly limited and that a lens with a photographic angle of view of about 45° may be used.

When the objective lens 30 and the rotating shaft 20a described earlier are viewed from a one-direction side in the Y direction, a position of the rotating shaft 20a and a position of the objective lens 30 coincide (the term "coincide" as used herein is intended to include the meaning of "substantially coincide"; the same applies hereinafter). With this configuration, the examination head 22 is rotated (swung) around the objective lens 30 by the swing rotation mechanism 20.

Respective illumination light sources 32 are provided at two end portions in the X direction (two left and right end portions) of the lens-barrel distal end face 28a, and two illumination light sources 32 are provided at a lower end portion of the lens-barrel distal end face 28a such that a camera 34a (to be described later) is sandwiched therebetween. Each illumination light source 32 is, for example, a Light Emitting Diode (LED) light source and illuminates the subject eye E.

The stereo camera 34 is used for alignment detection that detects relative positions in the X, Y, and Z directions of the subject eye E to the examination head 22. The stereo camera 34 includes a plurality of cameras 34a. For example, in the present embodiment, the stereo camera 34 includes two cameras 34a provided at the two end portions in the X direction (the two left and right end portions) corresponding to positions on the left and right of the objective lens 30 in the lens-barrel distal end face 28a and one camera 34a provided at the lower end portion corresponding to a position below the objective lens 30 in the lens-barrel distal end face 28a, three cameras 34a in total. The cameras 34a simultaneously photograph an anterior eye part of the subject eye E from a plurality of (three in the present embodiment) directions different from each other at the time of the alignment detection and output a plurality of (three) anterior eye part images of the subject eye E to the control device 40 (see Figure 4). The number of cameras 34a may be two, or four or more.

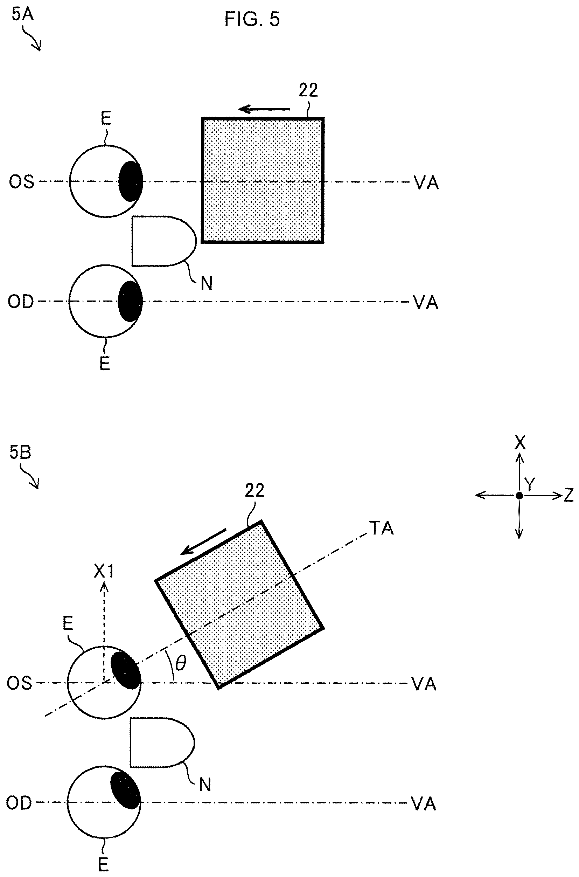

Positions of the cameras 34a may be appropriately changed. For example, if the camera 34a is provided in an upper region F1 (see Figure 2) above (in the Y direction) the two end portions in the X direction in the lens-barrel distal end face 28a, a pupil of the subject eye E may be hidden by upper lashes of the subject in photographing the subject eye E by the camera 34a. Additionally, if respective cameras 34a are provided in left and right lower oblique regions F2 (see Figure 2) between the two end portions in the X direction and the lower end portion in the lens-barrel distal end face 28a, each camera 34a is likely to come closer to a subject's nose N (see Figure 5) during alignment of the examination head 22. For this reason, the cameras 34a are preferably provided at the two end portions in the X direction and the lower end portion of the lens-barrel distal end face 28a.

Figure 4 is a block diagram illustrating a configuration of the ophthalmic device 10 according to the first embodiment. As illustrated in Figure 4, the ophthalmic device 10 includes a vision fixation light emitting unit 36, a display unit 37, a manipulation unit 38, a storage unit 39, and the control device 40 in addition to the XZ movement mechanism 16, the Y movement mechanism 18, the swing rotation mechanism 20, the examination head 22, and the stereo camera 34 described earlier.

The vision fixation light emitting unit 36 guides and fixes an eye direction of the subject eye E by emitting vision fixation light (a bright spot image) toward the subject eye E. The vision fixation light emitting unit 36 includes a publicly known vision fixation target display unit, a plurality of vision fixation holes, and an external fixation lamp (all not illustrated) (see Patent Literature 2 described above).

The vision fixation target display unit is provided inside the examination head 22 and is used for internal vision fixation that projects vision fixation light (e.g., a bright spot image) onto the subject eye E through the objective lens 30. The vision fixation holes are provided at a front surface in the Z direction (which may be the lens-barrel distal end face 28a) of the examination head 22 so as to surround the objective lens 30 and are used for peripheral vision fixation. The peripheral vision fixation is a vision fixation method for selectively lighting the vision fixation holes, thereby causing the subject eye E to make a great circumnutation in a desired direction. The external fixation lamp is provided at the face support 14 or the examination head 22 and is used for external vision fixation. The external vision fixation is a vision fixation method for causing the subject eye E to make a circumnutation in an arbitrary direction or make a greater circumnutation than under internal vision fixation by adjusting a light source position of the external fixation lamp, or adjusting an orientation of the subject eye E by guiding a visual line of the subject eye E or a fellow eye when the internal vision fixation cannot be performed.

As the display unit 37, which is a type of display device, for example, a touch-panel monitor is used. The display unit 37 displays screens such as a setup screen for the ophthalmic device 10, a manipulation screen (User Interface (UI) screen) for the ophthalmic device 10, anterior eye part images of the subject eye E photographed by the stereo camera 34, a result (a fundus image and a tomographic image of the subject eye E) of examining the subject eye E by the examination head 22.

Examples of the manipulation unit 38 include a publicly known manipulation lever, switches and a manipulation screen to be displayed on the display unit 37 (all not illustrated). The manipulation unit 38 is used to input an instruction such as a positional adjustment of the chin rest 14a and the forehead rest 14b, an XYZ movement and a rotation of the examination head 22, selecting the type of examination (from fundus imaging and OCT imaging), switching between automatic alignment and manual alignment, an examination start or saving an examination result (a fundus image or a tomographic image).

The storage unit 39 is a recording medium (storage medium) which stores a program to be executed by the control device 40, and various publicly known storages are used as the storage unit 39. A fundus image of the subject eye E photographed by the fundus camera unit 24 and a tomographic image of the subject eye E obtained through OCT imaging by the OCT unit 26 are saved in the storage unit 39.

The control device 40 performs overall control on the action of the units of the ophthalmic device 10 and executes the control such as alignment of the examination head 22 with the subject eye E, imaging of the fundus of the subject eye E by the fundus camera unit 24, and OCT imaging of the subject eye E by the OCT unit 26.

The control device 40 includes an arithmetic circuit including various processors and memories. Examples of the various processors include a Central Processing Unit (CPU), a Graphics Processing Unit (GPU), an Application Specific Integrated Circuit (ASIC), programmable logic devices (e.g., a Simple Programmable Logic Devices (SPLD), a Complex Programmable Logic Device (CPLD), and a Field Programmable Gate Arrays (FPGA)). Various functions of the control device 40 may be implemented by one processor or may be implemented by a plurality of processors of the same type or of different types.

The control device 40 functions as a nose photographing controlling unit 41A, a nose position detecting unit 41B, a tilting angle determining unit 43, an alignment detection unit 44, a drive controlling unit 46, a vision fixation controlling unit 48, a measurement controlling unit 50, a saving controlling unit 52, a retraction controlling unit 54, and a left-right eye switching controlling unit 56 by executing a control program stored in the storage unit 39.

Figure 5 is an explanatory diagram for explaining a method for automatic alignment of the examination head 22. Here, reference character "OD" in Figure 5 denotes a right eye (oculus dexter) and that reference character "OS" denotes a left eye (oculus sinister). In the present embodiment, the left eye OS (corresponding to a first eye according to the presently disclosed subject matter) that is one of left and right subject eyes E is first examined, and the right eye OD (corresponding to a second eye according to the presently disclosed subject matter) that is the other of the left and right subject eyes E is then examined.

As described earlier, in the ophthalmic device 10 (the fundus camera and the OCT device) supporting wide-angle photographing, an operating distance of the examination head 22 is short due to the objective lens 30 of large size. For this reason, as indicated by reference character 5A in Figure 5, when the ophthalmic device 10 moves the examination head 22 from a position in front of the subject eye E (the left eye OS here) toward the front side in the Z direction at the time of the automatic alignment of the examination head 22, the examination head 22 may come closer to the subject's nose N.

For the above-described reason, as indicated by reference character 5B in Figure 5, when viewed from the one-direction side in the Y direction, the ophthalmic device 10 according to the present embodiment brings the examination head 22 closer to the subject eye E from an oblique direction during the automatic alignment of the examination head 22.

Specifically, assume that an axis along the eye direction (an eye direction of the subject eye E observing infinite distance) of the subject eye E parallel to the Z direction is a reference axis VA, that for X direction, the outward direction away from the nose (N), based on the subject eye E (here, the left eye OS), is defined as X1. Furthermore, an axis obtained by tilting the reference axis VA in the outward direction X1 by a tilting angle θ around the subject eye E is designated as an axis TA of tilt. The examination head 22 is displaced to an examination position where examination (fundus imaging and OCT imaging) of the subject eye E is executable (hereinafter simply referred to as the examination position) along the axis TA of tilt when viewed from the one-direction side in the Y direction. The term "displacement" here subsumes movement in the X, Y, and Z directions and rotation of the examination head 22.

Referring back to Figure 4, the nose photographing controlling unit 41A drives the XZ movement mechanism 16 and the Y movement mechanism 18 to move the examination head 22 to a photographing position where the nose N is photographable by a plurality of (at least two here) cameras 34a corresponding to a photographing unit according to the presently disclosed subject matter before the start of the automatic alignment of the examination head 22, for example, when the examiner instructs a manipulation of starting examination on the subject eye E with the manipulation unit 38.

For example, the nose photographing controlling unit 41A estimates a position (three-dimensional position coordinates) of the subject's nose N based on positions in the Y direction of the chin rest 14a and the forehead rest 14b. Alternatively, the nose photographing controlling unit 41A causes the cameras 34a to execute photographing, and analyzes respective photographed images from the cameras 34a and detects positions of facial parts (e.g., the subject eye E, the nose N, a mouth, and eyebrows) of the subject, thereby estimating the position of the nose N. The nose photographing controlling unit 41A then detects a photographing position where the nose N is photographable by a plurality of cameras 34a based on a result of the estimation of the position of the nose N and known photographing conditions (a photographic angle of view, and a direction of a photographing optical axis) of each camera 34a. The nose photographing controlling unit 41A drives the XZ movement mechanism 16 and the Y movement mechanism 18 based on a result of the detection of the photographing position to positionally adjust the examination head 22 to the photographing position.

The nose position detecting unit 41B detects the position (three-dimensional coordinates) of the nose N based on respective photographed images of the nose N photographed by a plurality of cameras 34a and outputs a result of the detection to the retraction controlling unit 54. Since a method for detecting relative positions of various objects using the stereo camera 34 is a publicly known technique (see, for example, Patent Literature 1), a specific description thereof will be omitted.

The tilting angle determining unit 43 determines the tilting angle θ of the axis TA of tilt with respect to the reference axis VA when viewed from the one-direction side in the Y direction, i.e., the tilting angle θ of the axis TA of tilt with respect to the reference axis VA in an XZ plane. For example, the tilting angle determining unit 43 determines, as the tilting angle θ, a value that an examiner selects from a plurality of angles (e.g., 10°, 15°, and 20°) with the manipulation unit 38 and outputs information on the tilting angle θ to the drive controlling unit 46. As will be described in a fourth embodiment (to be described later), the tilting angle determining unit 43 may detect a relative position of the nose N to the examination head 22 based on photographed images obtained through stereoscopic photographing of the nose N by the stereo camera 34 and determine the tilting angle θ that can avoid the examination head 22 coming close to the nose N based on a result of the detection.

The alignment detection unit 44 detects a relative position of the subject eye E to the examination head 22 by identification of a pupil center position of the subject eye E and computation of three-dimensional coordinates of the pupil center position based on anterior eye part images of the subject eye E stereoscopically photographed by the cameras 34a of the stereo camera 34 during the automatic alignment of the examination head 22. Since a method for alignment detection using the stereo camera 34 is a publicly known technique (see Patent Literature 1 described above), a specific description thereof will be omitted.

The drive controlling unit 46 drives the XZ movement mechanism 16, the Y movement mechanism 18, and the swing rotation mechanism 20 to execute alignment of the examination head 22 with the subject eye E and switching of the subject eye E under examination (left-right eye switching). The alignment of the examination head 22 includes automatic alignment that is performed by automatically driving the XZ movement mechanism 16, the Y movement mechanism 18, and the swing rotation mechanism 20 and manual alignment that drives the XZ movement mechanism 16, the Y movement mechanism 18, and the swing rotation mechanism 20 in accordance with a manipulation input to the manipulation unit 38. The manipulation unit 38 is configured to execute switching between the automatic alignment and the manual alignment.

The drive controlling unit 46 determines the axis TA of tilt corresponding to the tilting angle θ based on the tilting angle θ initially determined by the tilting angle determining unit 43 at the time of the automatic alignment.

For example, the drive controlling unit 46 identifies the reference axis VA based on photographed images obtained through initial stereoscopic photographing of the face (e.g., the subject eye E or the nose N) of the subject by the stereo camera 34. Alternatively, the drive controlling unit 46 estimates the reference axis VA based on the positions in the Y direction of the chin rest 14a and the forehead rest 14b and identification information on the left eye OS and the right eye OD that are already known. The drive controlling unit 46 determines, as the axis TA of tilt, an axis obtained by tilting the reference axis VA in the outward direction X1 by the tilting angle θ around the subject eye E.

The drive controlling unit 46 then drives the XZ movement mechanism 16, the Y movement mechanism 18, and the swing rotation mechanism 20 based on the axis TA of tilt to start automatic alignment that automatically displaces the examination head 22 from an initial position when the ophthalmic device 10 is powered on to the examination position.

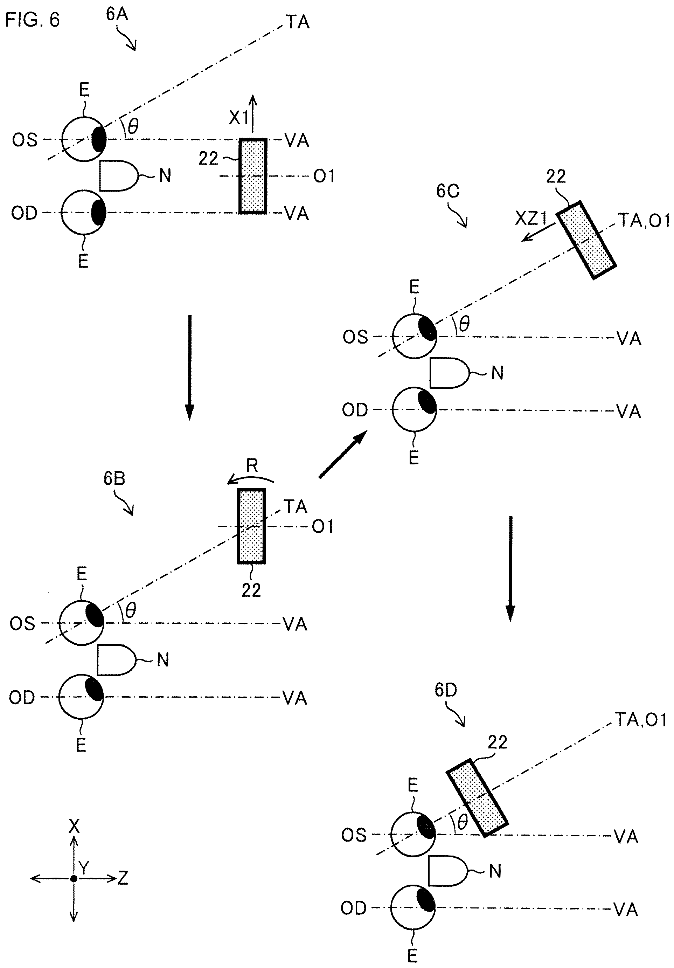

Figure 6 is an explanatory diagram for explaining an example 1-1 of the automatic alignment of the examination head 22 according to the first embodiment. As indicated by reference character 6A in Figure 6, the examination head 22 is initially arranged at a position where the optical axis O1 of the objective lens 30 is between the reference axes VA of the left and right subject eyes E (the left eye OS and the right eye OD) when viewed from the one-direction side in the Y direction, i.e., a position facing (the term "face" as used herein is intended to include the meaning of "substantially face"; the same applies hereinafter) the nose N.

First, the drive controlling unit 46 drives the XZ movement mechanism 16 to execute a first driving process of moving the examination head 22 from the initial position to the axis TA of tilt in the outward direction X1 when the examination head 22 is viewed from the one-direction side in the Y direction.

As indicated by reference character 6B in Figure 6, the drive controlling unit 46 drives the swing rotation mechanism 20 after completion of the first driving process to execute a second driving process of rotating the examination head 22 around the rotating shaft 20a by the tilting angle θ (see an arrow R). With this process, as indicated by reference character 6C in Figure 6, the optical axis O1 of the examination head 22 (the objective lens 30) becomes parallel to the axis TA of tilt. The second driving process may be executed before the first driving process.

The drive controlling unit 46 then drives the XZ movement mechanism 16 after completion of the second driving process to start a third driving process of moving the examination head 22 to the examination position along the axis TA of tilt when the examination head 22 is viewed from the one-direction side in the Y direction (see an arrow XZ1). With this process, the examination head 22 is moved toward the subject eye E while keeping the tilting angle θ constant (the term "constant" as used herein is intended to include the meaning of "substantially constant"; the same applies hereinafter).

Halfway through the automatic alignment, the examination head 22 is displaced to a position where the alignment detection unit 44 can identify the pupil center position of the subject eye E, i.e., a position where the alignment detection is possible. For this reason, an alignment detection result is input from the alignment detection unit 44 to the drive controlling unit 46. Y-direction positional adjustment of the examination head 22 by the Y movement mechanism 18 may be executed before the alignment detection (at any stage from the first driving process to the third driving process) such that the alignment detection by the alignment detection unit 44 is possible.

As indicated by reference character 6D in Figure 6, the drive controlling unit 46 drives the XZ movement mechanism 16 and the Y movement mechanism 18 based on an alignment detection result input from the alignment detection unit 44 to continue the third driving process until the examination head 22 reaches the examination position. In the third driving process that is executed based on the alignment detection result, the Y-direction positional adjustment of the examination head 22 is also executed.

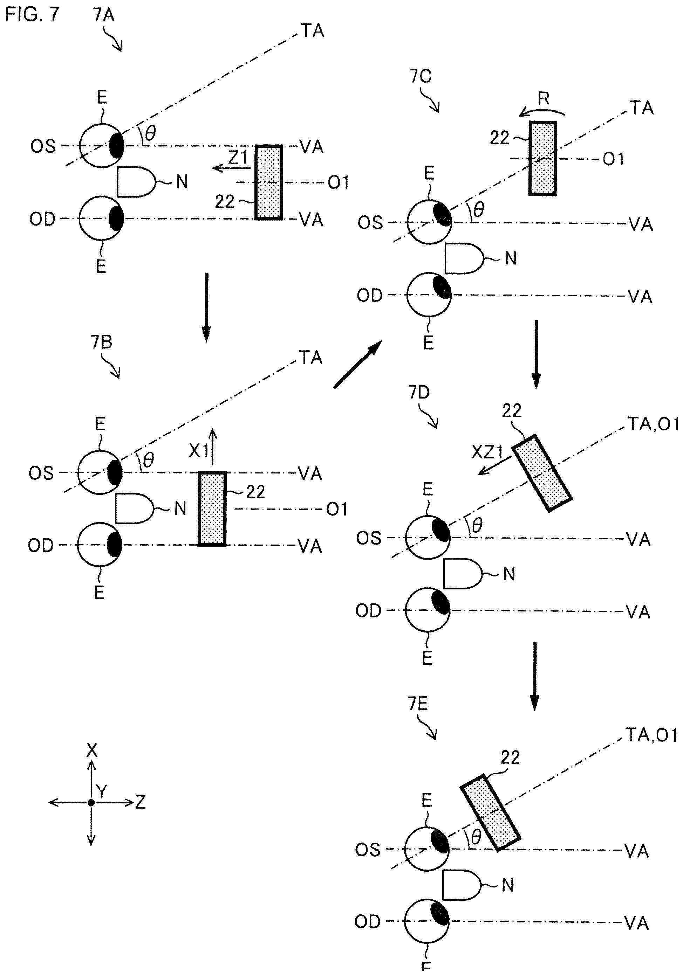

Figure 7 is an explanatory diagram for explaining an example 1-2 of the automatic alignment of the examination head 22 according to the first embodiment. The drive controlling unit 46 drives the XZ movement mechanism 16 to execute a first driving process of first moving the examination head 22 toward the front side (the subject eye E side) in the Z direction by a predetermined distance (see an arrow Z1), as indicated by reference character 7A in Figure 7. The drive controlling unit 46 then moves the examination head 22 to the axis TA of tilt in the outward direction X1, as indicated by reference character 7B in Figure 7. A distance of movement of the examination head 22 toward the front side in the Z direction is not particularly limited as long as a safe distance can be secured between the examination head 22 and the nose N. For example, the distance of movement may be determined based on a result of computing a Z-direction distance from the examination head 22 to the nose N based on photographed images obtained through stereoscopic photographing of the nose N by the stereo camera 34.

Moving the examination head 22 first toward the front side in the Z direction and then in the outward direction X1 to the tilt axis TA can reduce a moving distance of the examination head 22 along the direction X1, compared with the example 1-1 illustrated in Figure 6 described earlier.

As indicated by reference character 7C in Figure 7, the drive controlling unit 46 drives the swing rotation mechanism 20 after completion of the first driving process to execute the same second driving process as the example 1-1 (see reference character 6B in Figure 6) and make the optical axis O1 parallel to the axis TA of tilt. The second driving process may be executed before the first driving process in the example 1-2 as well.

As indicated by reference character 7D in Figure 7, the drive controlling unit 46 drives the XZ movement mechanism 16 after completion of the second driving process to execute a third driving process in the same manner as the example 1-1 (see reference characters 6C and 6D in Figure 6), thereby moving the examination head 22 to the examination position along the axis TA of tilt when the examination head 22 is viewed from the one-direction side in the Y direction. As indicated by reference character 7E in Figure 7, the drive controlling unit 46 drives the XZ movement mechanism 16 and the Y movement mechanism 18 based on alignment detection performed by the alignment detection unit 44 halfway through the automatic alignment to continue the third driving process until the examination head 22 reaches the examination position.

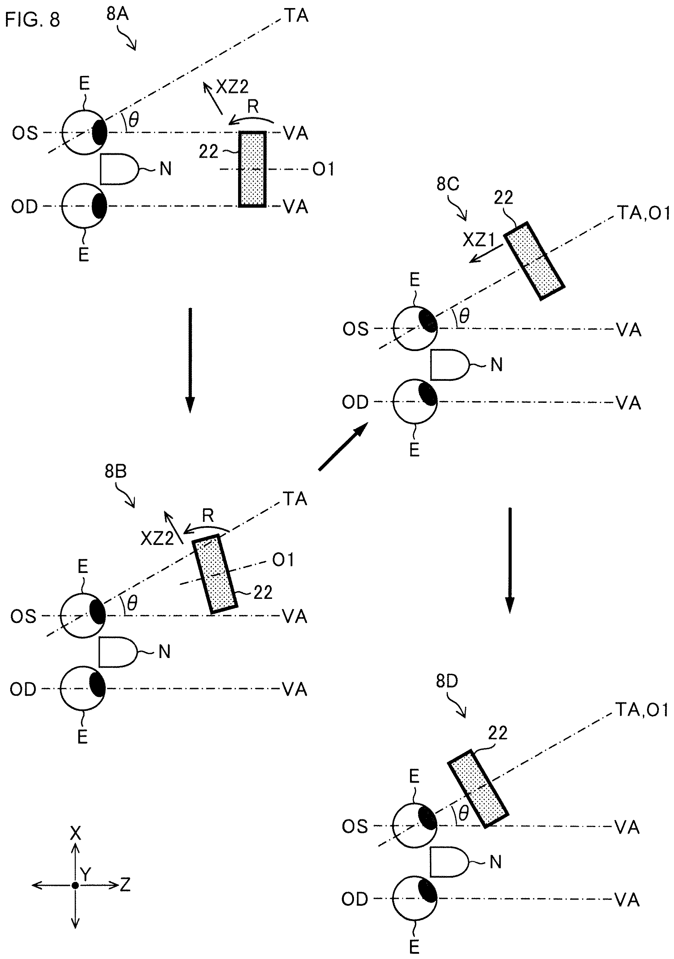

Figure 8 is an explanatory diagram for explaining an example 1-3 of the automatic alignment of the examination head 22 according to the first embodiment. As indicated by reference characters 8A and 8B in Figure 8, the drive controlling unit 46 drives the XZ movement mechanism 16, the Y movement mechanism 18, and the swing rotation mechanism 20 to execute a first driving process of simultaneously executing movement of the examination head 22 toward the front side in the Z direction and in the outward direction X1 and rotation of the examination head 22 by the tilting angle θ (see an arrow XZ2 and the arrow R). With this process, it is possible to move the examination head 22 to the axis TA of tilt in an oblique direction when the examination head 22 is viewed from the one-direction side in the Y direction and make the optical axis O1 parallel to the axis TA of tilt.

In the first driving process described in the example 1-3, the examination head 22 may be displaced to a position on the axis TA of tilt where the stereo camera 34 can photograph the anterior eye part of the subject eye E or to a position where an observation optical system (not illustrated) inside the examination head 22 can photograph the anterior eye part of the subject eye E via a shortest route.

As indicated by reference character 8C in Figure 8, the drive controlling unit 46 drives the XZ movement mechanism 16 and the Y movement mechanism 18 after completion of the first driving process to execute a second driving process. The second driving process in the example 1-3 is the same process as the third driving processes in the example 1-1 and the example 1-2, and moves the examination head 22 to the examination position along the axis TA of tilt when the examination head 22 is viewed from the one-direction side in the Y direction. As indicated by reference character 8D in Figure 8, the drive controlling unit 46 drives the XZ movement mechanism 16 and the Y movement mechanism 18 based on alignment detection performed by the alignment detection unit 44 halfway through the automatic alignment to continue the second driving process until the examination head 22 reaches the examination position.

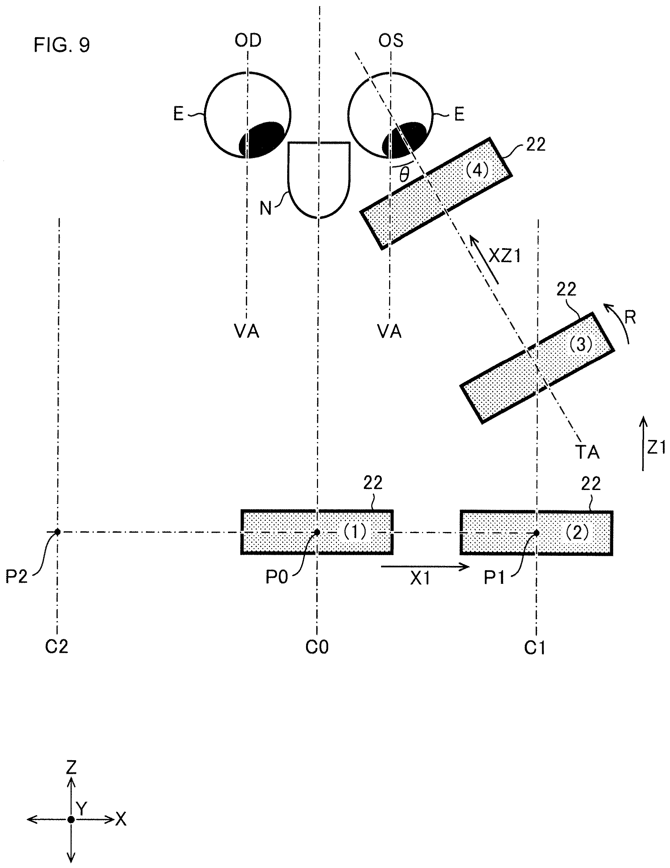

Figure 9 is an explanatory diagram for explaining an example 1-4 of the automatic alignment of the examination head 22 according to the first embodiment. Here, reference character P0 in Figure 9 denotes a poweron or startup initial position which is the initial position for the examination head 22 when the ophthalmic device 10 is powered on. Reference character C0 in Figure 9 denotes a centerline which passes through the poweron initial position P0 and is parallel to the Z direction. The optical axis O1 of the examination head 22 is not illustrated in Figure 9 for the sake of brevity.

As indicated by the parenthesized number (1) in Figure 9, the examination head 22 at the poweron initial position P0 is arranged at a position facing the nose N. The drive controlling unit 46 first drives the XZ movement mechanism 16 to execute a first driving process of moving the examination head 22 from the poweron initial position P0 to a pre-examination initial position P1 determined in advance in the outward direction X1 when the examination head 22 is viewed from the one-direction side in the Y direction, as indicated by the parenthesized number (2). When the subject eye E (the first eye) is the right eye OD, the drive controlling unit 46 drives the XZ movement mechanism 16 to move the examination head 22 from the poweron initial position P0 to a pre-examination initial position P2 determined in advance when the examination head 22 is viewed from the one-direction side in the Y direction.

The pre-examination initial position P1 corresponds to a first initial position according to the presently disclosed subject matter and is, for example, a position where the stereo camera 34 or the observation optical system (not illustrated) inside the examination head 22 can photograph an anterior eye part of the left eye OS. The pre-examination initial position P2 corresponds to a second initial position according to the presently disclosed subject matter and is, for example, a position where the stereo camera 34 or the observation optical system can photograph an anterior eye part of the right eye OD. Here, reference character C1 in Figure 9 denotes a straight line passing through the pre-examination initial position P1 and parallel to the Z direction, and reference character C2 denotes a straight line passing through the pre-examination initial position P2 and parallel to the Z direction.

The pre-examination initial positions P1 and P2 are set to be spaced in the X direction when viewed from the one-direction side in the Y direction, and an intermediate position between the two positions in the X direction coincides with the poweron initial position P0 (the centerline C0). The poweron initial position P0 may be shifted from the pre-examination initial positions P1 and P2 in the Z direction.

The drive controlling unit 46 drives the XZ movement mechanism 16 after completion of the first driving process to execute a second driving process. The second driving process in the example 1-4 is a process of moving the examination head 22 toward the front side in the Z direction (see the arrow Z1) from the pre-examination initial position P1 to the axis TA of tilt when the examination head 22 is viewed from the one-direction side in the Y direction and then making the optical axis O1 parallel to the axis TA of tilt by driving the swing rotation mechanism 20 to rotate the examination head 22 (see the arrow R), as indicated by the parenthesized number (3).

The drive controlling unit 46 executes the same third driving process as the example 1-1 (see reference characters 6C and 6D in Figure 6) to move the examination head 22 to an examination position along the axis TA of tilt when the examination head 22 is viewed from the one-direction side in the Y direction, as indicated by the parenthesized number (4). The drive controlling unit 46 drives the XZ movement mechanism 16 and the Y movement mechanism 18 based on a result of alignment detection by the alignment detection unit 44 halfway through the automatic alignment to continue the third driving process until the examination head 22 reaches the examination position.

Referring back to Figure 4, the vision fixation controlling unit 48 causes the vision fixation light emitting unit 36 to emit vision fixation light at least during a period from before the start of the automatic alignment of the examination head 22 to completion of examination on the subject eye E by the examination head 22. This makes it possible to guide and fix an eye direction of the subject to a direction of the vision fixation light during movement of the examination head 22 to the examination position at the time of the automatic alignment. For this reason, the subject eye E can be made to circumnutate so as to follow the movement of the examination head 22. As a result, the eye direction of the subject eye E can be kept fixed to the examination head 22.

The measurement controlling unit 50 controls fundus photographing of the subject eye E by the fundus camera unit 24 and photographing of a tomographic image of the subject eye E by the OCT unit 26. For example, the measurement controlling unit 50 drives a focus optical system (not illustrated) (see Patent Literature 1) housed in the examination head 22 after completion of the automatic alignment of the examination head 22 to execute an autofocusing process of focusing the examination head 22 on a part to be observed (e.g., the fundus) of the subject eye E. The measurement controlling unit 50 then executes fundus photographing of the subject eye E by the fundus camera unit 24 or OCT imaging of the subject eye E by the OCT unit 26.

The measurement controlling unit 50 acquires a fundus image of the subject eye E from the fundus camera unit 24 and outputs the fundus image to the saving controlling unit 52 when fundus photographing of the subject eye E by the fundus camera unit 24 is executed. The measurement controlling unit 50 generates a tomographic image of the subject eye E based on a signal such as a detection signal output from the OCT unit 26 by a publicly known method and outputs the tomographic image to the saving controlling unit 52 when OCT imaging of the subject eye E by the OCT unit 26 is executed.

The saving controlling unit 52 causes the display unit 37 to display a fundus image or a tomographic image of the subject eye E input from the measurement controlling unit 50. The saving controlling unit 52 saves the fundus image or the tomographic image of the subject eye E in the storage unit 39 when the examiner inputs an image saving manipulation to the manipulation unit 38.

The retraction controlling unit 54 operates after completion of examination on the left eye OS (the first eye) by the examination head 22, i.e., completion of photographing or imaging of the left eye OS by the fundus camera unit 24 or the OCT unit 26. In the ophthalmic device 10 (the examination head 22) supporting wide-angle photographing, an operating distance between the left eye OS and the examination head 22 is short. For this reason, when the subject moves the face after completion of the examination on the left eye OS by the examination head 22 or at the time of left-right eye switching (to be described later), the examination head 22 may come close to the face of the subject, the nose N in the face to be specific.

For the above-described reason, the retraction controlling unit 54 drives at least one of the XZ movement mechanism 16, the Y movement mechanism 18, and the swing rotation mechanism 20 after completion of the examination on the left eye OS to execute retraction control that retracts the examination head 22 in a direction away from the face, the nose N to be specific, of the subject. At this time, the retraction controlling unit 54 determines a retraction direction (the X, Y, and Z directions and a rotation direction) of moving the examination head 22 away from the nose N based on a result of detection by the nose position detecting unit 41B and retracts the examination head 22 in the retraction direction.

The retraction direction for the examination head 22 may be fixed. In this case, photographing of the nose N by the stereo camera 34 and position detection of the nose N by the nose position detecting unit 41B before the start of the automatic alignment can be omitted.

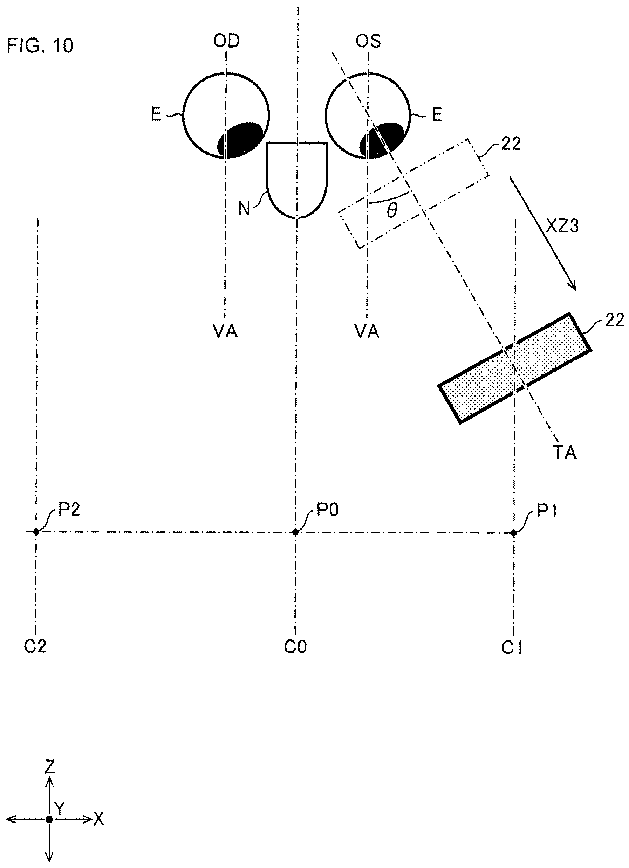

Figure 10 is an explanatory diagram for explaining an example 1 of the retraction control of the examination head 22. As illustrated in Figure 10, the retraction controlling unit 54 drives the XZ movement mechanism 16 to move the examination head 22 in a direction (see an arrow XZ3) away from the nose N along the axis TA of tilt.

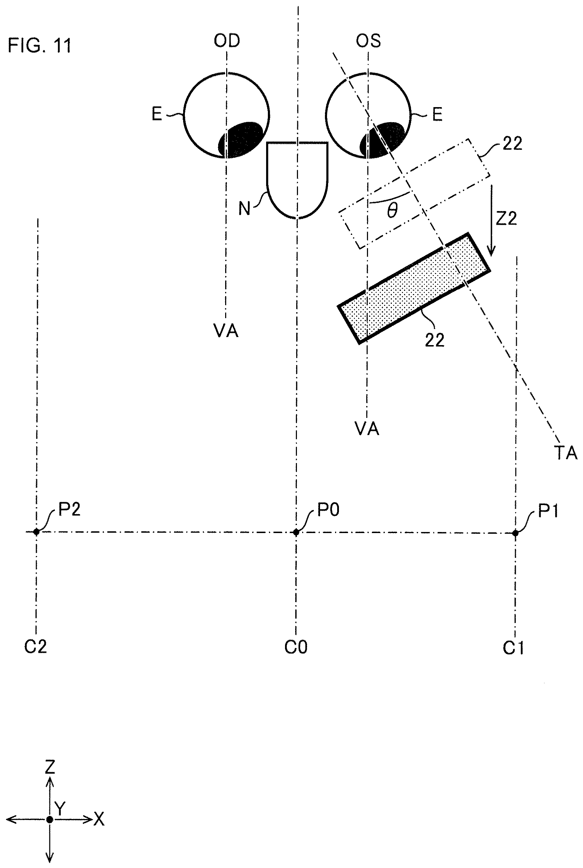

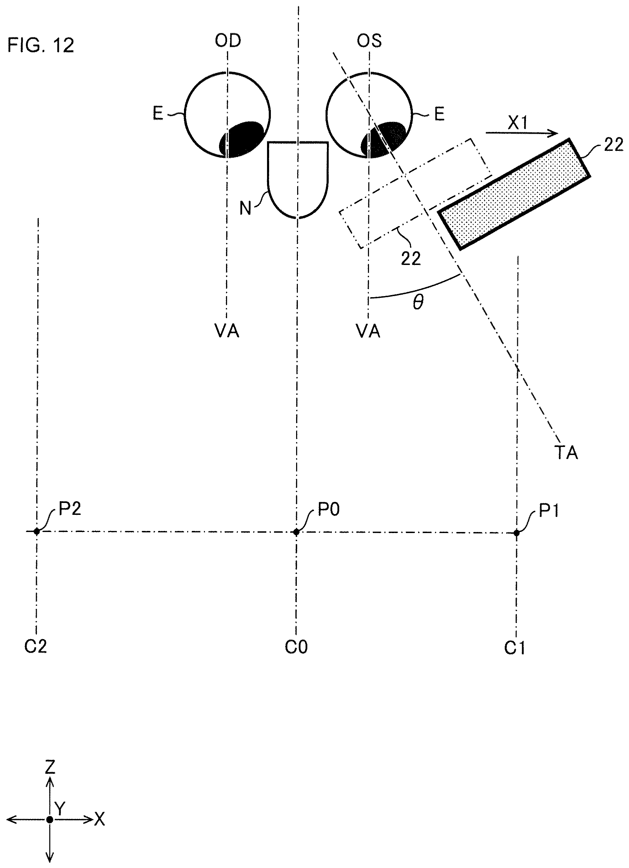

Figure 11 is an explanatory diagram for explaining an example 2 of the retraction control of the examination head 22. Figure 12 is an explanatory diagram for explaining an example 3 of the retraction control of the examination head 22. The retraction controlling unit 54 drives the XZ movement mechanism 16 to move the examination head 22 toward a rear side in the Z direction (a rearward direction Z2), as illustrated in Figure 11, or to move the examination head 22 in the outward direction X1, as illustrated in Figure 12, thereby moving the examination head 22 away from the nose N. The examination head 22 may be moved simultaneously in the rearward direction Z2 and in the outward direction X1, i.e., in an oblique direction (including a direction different from the axis TA of tilt).

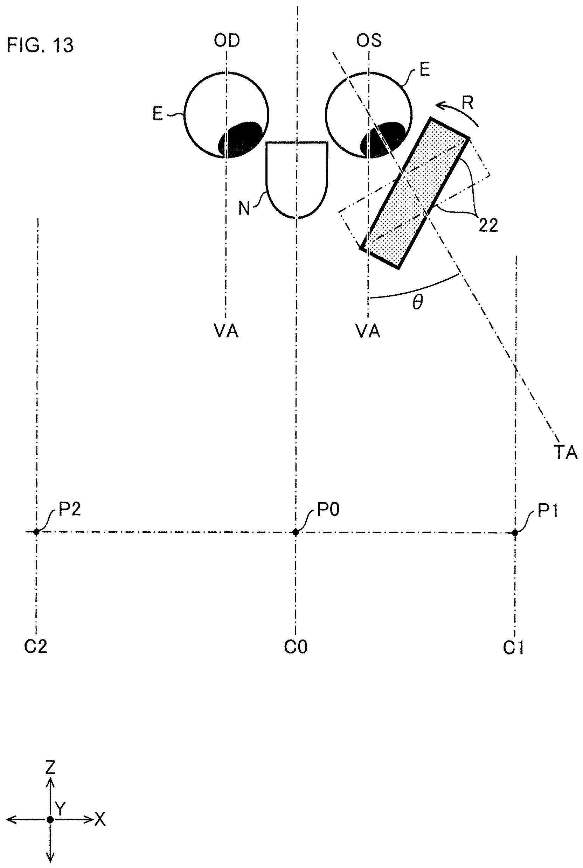

Figure 13 is an explanatory diagram for explaining an example 4 of the retraction control of the examination head 22. As illustrated in Figure 13, the retraction controlling unit 54 drives the swing rotation mechanism 20 to rotate the examination head 22 in a direction (see the arrow R) away from the nose N. A rotation direction for the examination head 22 which moves the examination head 22 away from the nose N can be determined based on information such as left and right eye information indicating whether the subject eye E is the left eye OS or the right eye OD, or how the outward direction X1 is oriented (see reference character 6A in Figure 6).

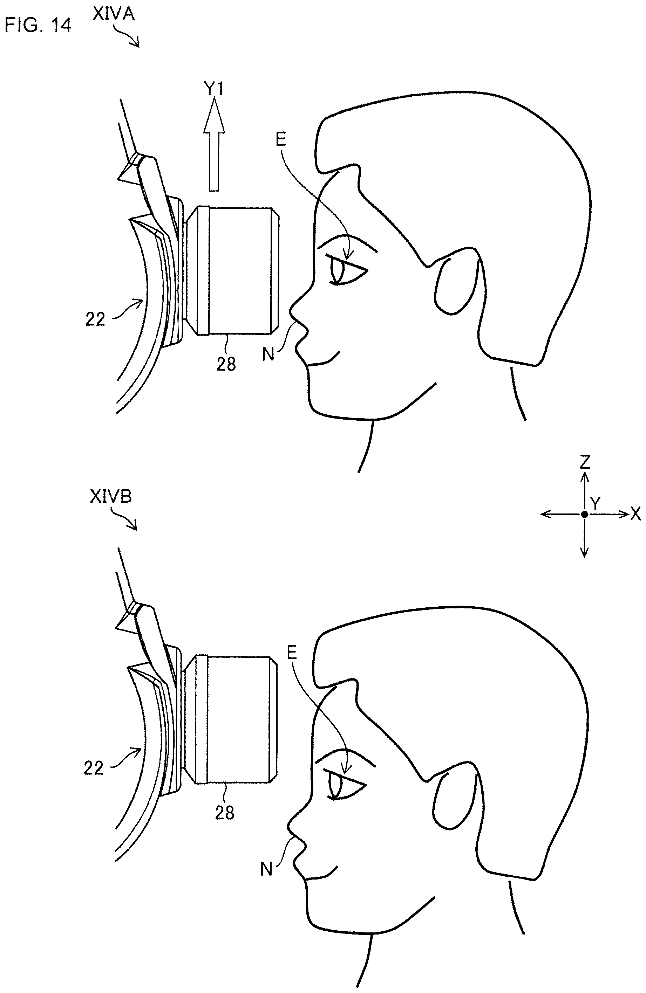

Figure 14 is an explanatory diagram for explaining an example 5 of the retraction control of the examination head 22. As indicated by reference characters XIVA and XIVB in Figure 14, the retraction controlling unit 54 drives the Y movement mechanism 18 to move the examination head 22 toward the upper side in the Y direction (an outward direction Y1), thereby moving the examination head 22 away from the nose N.

The example 1 to the example 5 described above of the retraction control may be appropriately performed in combination.

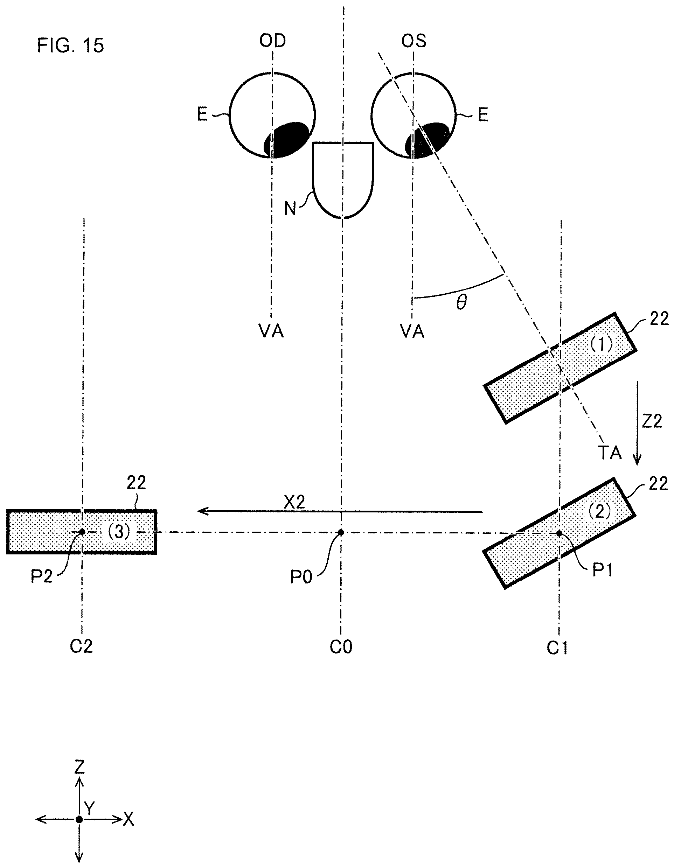

Figure 15 is an explanatory diagram for explaining an example of a left-right eye switching controlling process to be executed by the left-right eye switching controlling unit 56. As illustrated in Figure 15 and Figure 4, the left-right eye switching controlling unit 56 drives at least the XZ movement mechanism 16 and the swing rotation mechanism 20 after completion of the retraction control (the example 1 illustrated in Figure 10) by the retraction controlling unit 54 to execute left-right eye switching control that displaces the examination head 22 to the pre-examination initial position P2 via the pre-examination initial position P1.

For example, the left-right eye switching controlling unit 56 drives the XZ movement mechanism 16 to move the examination head 22 after retraction control indicated by the parenthesized number (1) to the pre-examination initial position P1 indicated by the parenthesized number (2). The left-right eye switching controlling unit 56 then drives the XZ movement mechanism 16 to execute a left-right movement process of moving the examination head 22 in a left-right switching direction X2 which is a direction opposite to the outward direction X1 from the pre-examination initial position P1 to the pre-examination initial position P2 indicated by the parenthesized number (3). The left-right eye switching controlling unit 56 also drives the swing rotation mechanism 20 before or after arrival of the examination head 22 at the pre-examination initial position P2 to execute a rotation process of rotating the examination head 22 such that the optical axis O1 is parallel to the reference axis VA (the Z direction).

The left-right eye switching controlling unit 56 may drive at least the XZ movement mechanism 16 and the swing rotation mechanism 20 after the retraction control by the retraction controlling unit 54 to displace the examination head 22 to the pre-examination initial position P2 via the pre-examination initial position P1. For example, the examination head 22 after the retraction control indicated by the parenthesized number (1) may be displaced to the pre-examination initial position P2 indicated by the parenthesized number (3) via a shortest route.

### [Function of Ophthalmic Device According to First Embodiment]

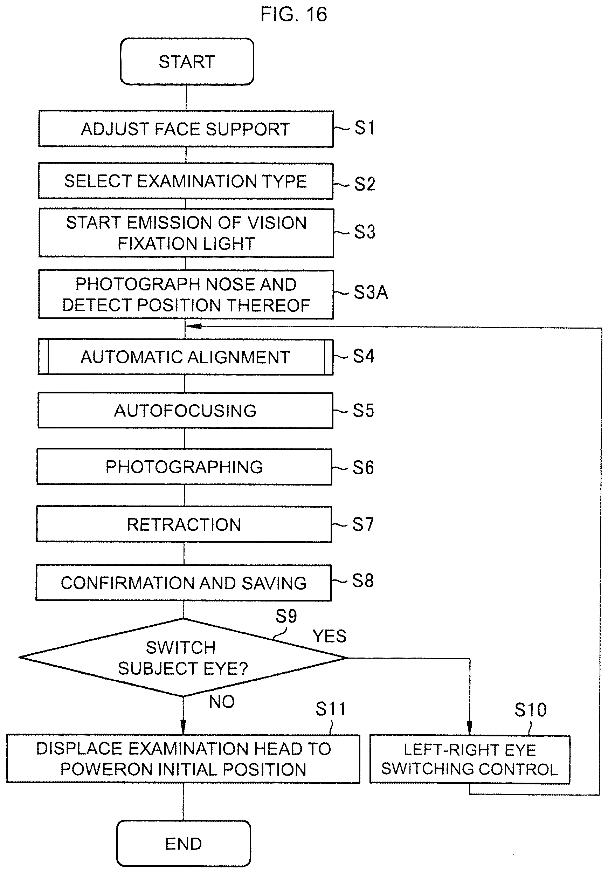

Figure 16 is a flowchart illustrating a flow of a process of examining the subject eye E by the ophthalmic device 10 according to the first embodiment with the above-described configuration. As illustrated in Figure 16, the examiner manipulates the manipulation unit 38 with the subject placing his/her chin against the chin rest 14a and his/her forehead against the forehead rest 14b to adjust height positions (positions in the Y direction) of the chin rest 14a and the forehead rest 14b to fit the subject (step S1).

The examiner then manipulates the manipulation unit 38 to select the type of examination on the subject eye E (fundus photographing by the fundus camera unit 24 or OCT imaging by the OCT unit 26) (step S2). The examiner also manipulates the manipulation unit 38 to select an automatic alignment mode as an alignment mode of the examination head 22. When the examination type of the subject eye E and the alignment mode are selected, the vision fixation controlling unit 48 causes the vision fixation light emitting unit 36 to emit vision fixation light (step S3). This allows guiding and fixation of the eye direction of the subject eye E.

When the examiner inputs an examination start manipulation to the manipulation unit 38, the nose photographing controlling unit 41A and the nose position detecting unit 41B operate first. The nose photographing controlling unit 41A drives the XZ movement mechanism 16 and the Y movement mechanism 18 to positionally adjust the examination head 22 to a photographing position for the nose N and then causes a plurality of cameras 34a to simultaneously photograph the nose N and each camera 34a to output a photographed image of the nose N to the nose position detecting unit 41B (step S3A). The nose position detecting unit 41B detects the position of the nose N based on respective photographed images of the nose N photographed by the plurality of cameras 34a and outputs a result of the detection to the retraction controlling unit 54 (step S3A).

The automatic alignment of the examination head 22 with the subject eye E is then executed (step S4).

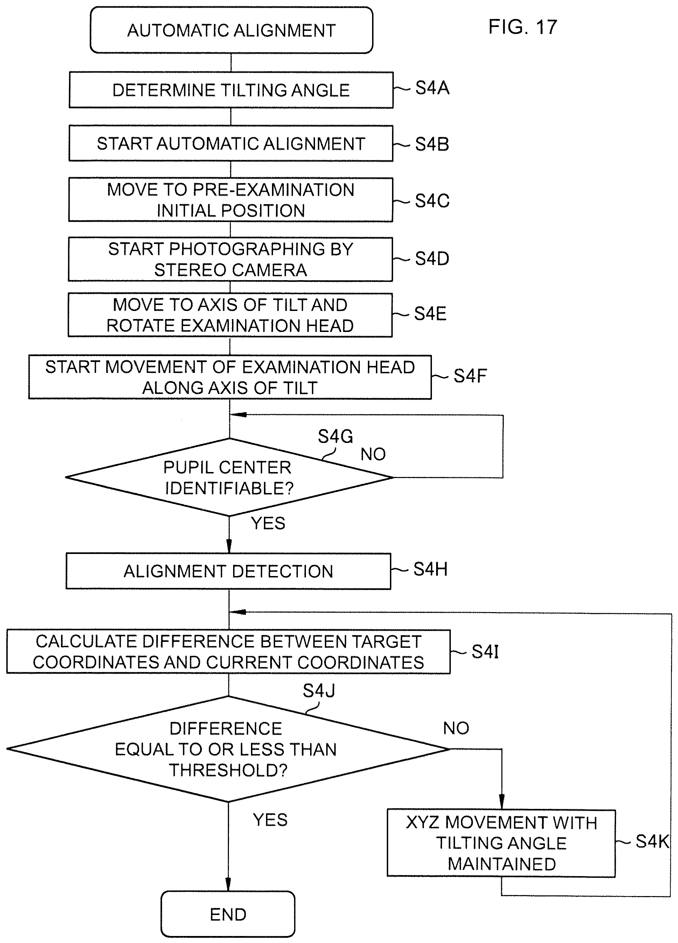

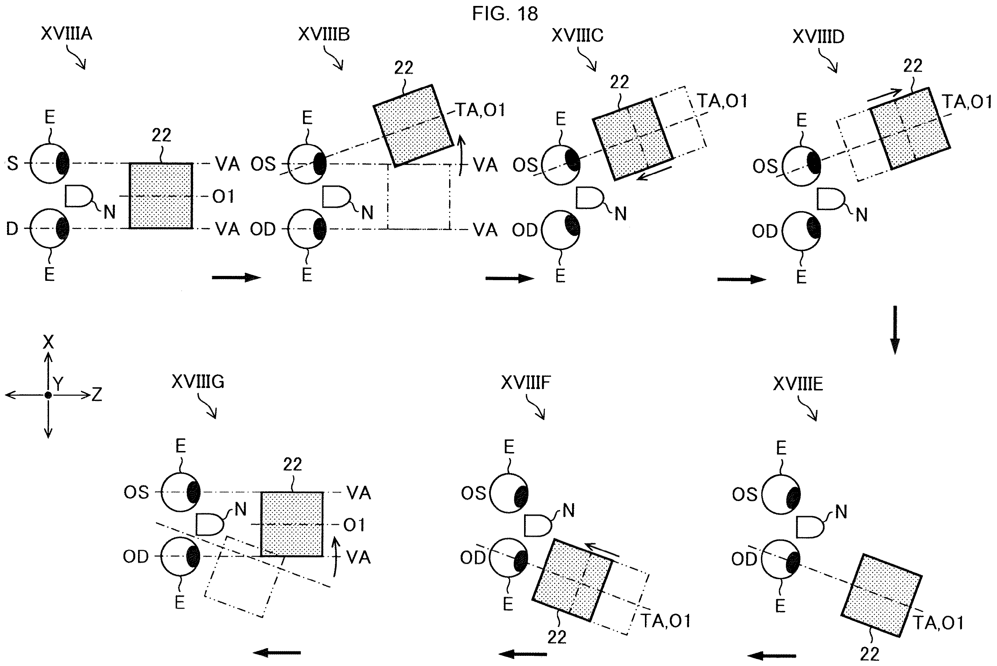

Figure 17 is a flowchart illustrating a flow of an automatic alignment process for the examination head 22 which pertains to a method for operating an ophthalmic device according to the presently disclosed subject matter. Figure 18 is an explanatory diagram for explaining displacement of the examination head 22 after the start of the automatic alignment. Here, an example of the automatic alignment in the example 1-4 illustrated in Figure 9 will be described below.

As illustrated in Figures 17 and 18, after the tilting angle determining unit 43 determines, as the tilting angle θ, an angle selected in advance with the manipulation unit 38 by the examiner, the tilting angle determining unit 43 outputs information on the tilting angle θ to the drive controlling unit 46 (step S4A). For this reason, after the drive controlling unit 46 determines the axis TA of tilt based on the tilting angle θ, the drive controlling unit 46 starts the automatic alignment of the examination head 22 (step S4B).

The drive controlling unit 46 drives the XZ movement mechanism 16 to execute a first driving process of moving the examination head 22 from the poweron initial position P0 to the pre-examination initial position P1 in the outward direction X1 when the examination head 22 is viewed from the one-direction side in the Y direction (step S4C). When the examination head 22 reaches the pre-examination initial position P1, the alignment detection unit 44 causes the cameras 34a of the stereo camera 34 to start photographing and continuously executes acquisition of photographed images from the cameras 34a and analysis of the photographed images (step S4D).

When the first driving process is completed, the drive controlling unit 46 drives the XZ movement mechanism 16 to move the examination head 22 toward the front side in the Z direction to the axis TA of tilt when the examination head 22 is viewed from the one-direction side in the Y direction and then drives the swing rotation mechanism 20 to execute a second driving process of rotating the examination head 22 to make the optical axis O1 parallel to the axis TA of tilt (step S4E). With this process, the examination head 22 is displaced from the poweron initial position P0 indicated by reference character XVIIIA in Figure 18 to the axis TA of tilt, as indicated by reference character XVIIIB, and the optical axis O1 becomes parallel to the axis TA of tilt. Note that emission of vision fixation light from the vision fixation light emitting unit 36 allows the eye direction of the subject eye E to follow displacement of the examination head 22.

The drive controlling unit 46 drives the XZ movement mechanism 16 to start a third driving process of moving the examination head 22 to the examination position along the axis TA of tilt when the examination head 22 is viewed from the one-direction side in the Y direction (step S4F, corresponding to a drive controlling step according to the presently disclosed subject matter), as indicated by reference character XVIIIC in Figure 18. The displacement of the examination head 22 to the examination position along the axis TA of tilt at the time of the automatic alignment prevents the examination head 22 from coming closer to the nose N.

While the first driving process to the third driving process are executed, the alignment detection unit 44 waits for a chance for alignment detection (NO in step S4G) until the pupil center position of the subject eye E can be identified from photographed images acquired from the cameras 34a. Halfway through the automatic alignment, the cameras 34a photograph the anterior eye part of the subject eye E, and anterior eye part images of the subject eye E are input as photographed images from the cameras 34a to the alignment detection unit 44. This allows the alignment detection unit 44 to identify the pupil center position of the subject eye E based on the anterior eye part images input from the cameras 34a (YES in step S4G).

The alignment detection unit 44 then executes alignment detection that detects the relative position of the subject eye E to the examination head 22 by converting the pupil center position of the subject eye E into three-dimensional coordinates (step S4H). The alignment detection unit 44 outputs a detection result of the alignment detection to the drive controlling unit 46.

The drive controlling unit 46 switches the alignment mode of the examination head 22 to a manual alignment mode when the alignment detection unit 44 is incapable of alignment detection during a period from the start of the automatic alignment or the start of the third driving process to movement of the examination head 22 for a fixed time determined in advance or over a fixed distance (the same applies to second and subsequent embodiments (to be described later)). This prevents the examination head 22 from coming closer to the subject eye E in a state where alignment detection is impossible.

The drive controlling unit 46 drives the XZ movement mechanism 16 and the Y movement mechanism 18 based on the alignment detection result input from the alignment detection unit 44 to continue the third driving process until the examination head 22 reaches the examination position. Specifically, the drive controlling unit 46 calculates a difference between three-dimensional coordinates (target coordinates) of the examination position determined based on the alignment detection result and three-dimensional coordinates of the current examination head 22 (current coordinates) and continues the third driving process until the difference is equal to or less than a threshold (step S4I, NO in step S4J, and step S4K). In this manner, the examination head 22 is moved to the examination position while maintaining the tilting angle θ.

When the difference between the target coordinates and the current coordinates is equal to or less than the threshold, the drive controlling unit 46 stops driving the XZ movement mechanism 16 and the Y movement mechanism 18 to end the automatic alignment (YES in step S4J).

Referring back to Figures 12 and 18, when the automatic alignment is completed, the measurement controlling unit 50 drives the focus optical system (not illustrated) to execute autofocusing (step S5). After that, the measurement controlling unit 50 executes fundus photographing of the left eye OS by the fundus camera unit 24 or OCT imaging of the left eye OS by the OCT unit 26 (step S6). The measurement controlling unit 50 outputs a fundus image of the left eye OS acquired from the fundus camera unit 24 to the saving controlling unit 52 when fundus photographing is executed. The measurement controlling unit 50 generates a tomographic image of the left eye OS based on a signal such as a detection signal output from the OCT unit 26 and outputs the tomographic image to the saving controlling unit 52 when OCT imaging is executed.

When the fundus photographing or OCT imaging of the left eye OS is completed, the retraction controlling unit 54 determines a retraction direction for the examination head 22 based on a result of detecting the position of the nose N by the nose position detecting unit 41B. The retraction controlling unit 54, for example, drives the XZ movement mechanism 16 to retract the examination head 22 in a direction away from the nose N along the axis TA of tilt (step S7, corresponding to a retraction controlling step according to the presently disclosed subject matter), as indicated by reference character XVIIID in Figure 18. The retraction controlling unit 54 may drive the XZ movement mechanism 16 to retract the examination head 22 toward the rear side in the Z direction or in the outward direction X1, drive the swing rotation mechanism 20 to rotate the examination head 22 in a direction away from the nose N, or drive the Y movement mechanism 18 to retract the examination head 22 toward the upper side in the Y direction. This prevents the examination head 22 from coming close to the nose N even if the face of the subject moves after completion of examination on the left eye OS.

When the fundus photographing or OCT imaging of the left eye OS is completed, the saving controlling unit 52 causes the display unit 37 to display the fundus image or the tomographic image of the subject eye E input from the measurement controlling unit 50. This allows the examiner to confirm whether a desired fundus image or tomographic image is obtained. When a desired fundus image or tomographic image is obtained, the examiner inputs an image saving manipulation to the manipulation unit 38. With this manipulation, the saving controlling unit 52 saves the fundus image or tomographic image of the subject eye E in the storage unit 39 (step S8).

After completion of the retraction of the examination head 22 by the retraction controlling unit 54, the left-right eye switching controlling unit 56 drives the mechanisms such as the XZ movement mechanism 16 and the swing rotation mechanism 20 to execute left-right eye switching control that displaces the examination head 22 to the pre-examination initial position P2 via the pre-examination initial position P1 (YES in step S9, and step S10), as illustrated in Figure 15.

When the left-right eye switching control is completed, the processes in step S4 (step S4D and the subsequent steps) to step S8 are repeatedly executed. In this case, the examination head 22 is displaced to the axis TA of tilt corresponding to the right eye OD (see reference character XVIIIE in Figure 18) and is further moved to an examination position for the right eye OD along the axis TA of tilt (see reference character XVIIIF in Figure 18), under control by the drive controlling unit 46. When examination on the right eye OD is completed, the examination head 22 is displaced from the examination position for the right eye OD to the poweron initial position P0 under control by the drive controlling unit 46 or the retraction controlling unit 54 (step S11), as indicated by reference character XVIIIG in Figure 18. The examination head 22 may be retracted from the nose N and be displaced to the poweron initial position P0 upon completion of the examination on the right eye OD, as illustrated in Figures 10 to 14.

As described above, in the ophthalmic device 10 according to the first embodiment, the examination head 22 is retracted from the face (the nose N) of the subject upon completion of the examination on the subject eye E (the first eye). This makes it possible to reliably avoid the examination head 22 coming close to the face, the nose N to be specific, even if motion occurs in the face of the subject during left-right eye switching control after completion of examination on one of the left and right subject eyes E.

### [Second Embodiment]

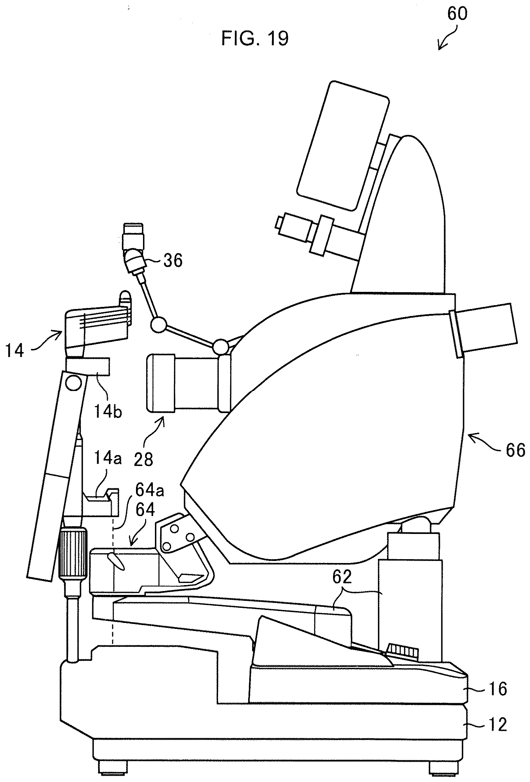

Figure 19 is a side view of an ophthalmic device 60 according to a second embodiment. While the examination head 22 is rotated (swung) around the objective lens 30 by the swing rotation mechanism 20 (the rotating shaft 20a) in the ophthalmic device 10 according to the above-described first embodiment, the ophthalmic device 60 according to the second embodiment includes an examination head 66 different in rotation center position from that in the first embodiment.

Components functionally or structurally identical to those in the ophthalmic device 10 according to the first embodiment are denoted by identical reference numerals, and a description thereof will be omitted.

As illustrated in Figure 19, the ophthalmic device 60 is a fundus camera and includes a base 12, a face support 14, an XZ movement mechanism 16, a vision fixation light emitting unit 36 (only an external fixation lamp of which is illustrated), a Y movement mechanism 62, a swing rotation mechanism 64, and the examination head 66. The ophthalmic device 60 also includes a stereo camera 34, a display unit 37, a manipulation unit 38, a storage unit 39, and a control device 40 (all not illustrated) described in the first embodiment.

The Y movement mechanism 62, together with the XZ movement mechanism 16, constitutes a movement mechanism according to the presently disclosed subject matter. The Y movement mechanism 62 has a shape extending toward a front side in a Z direction. The swing rotation mechanism 64 is provided at a distal end portion on the front side in the Z direction of the Y movement mechanism 62. The Y movement mechanism 62 integrally moves the swing rotation mechanism 64 and the examination head 66 in a Y direction. The XZ movement mechanism 16 and the Y movement mechanism 62 are capable of integrally moving the swing rotation mechanism 64 and the examination head 66 in an X direction and the Y and Z directions.

The swing rotation mechanism 64 corresponds to a rotation mechanism according to the presently disclosed subject matter and, together with the XZ movement mechanism 16 and the Y movement mechanism 62, constitutes a displacement mechanism according to the presently disclosed subject matter. The swing rotation mechanism 64 has a rotation axis 64a parallel to the Y direction and rotates the examination head 66 around the rotation axis 64a. The rotation axis 64a is provided in front of a lens-barrel 28 (an objective lens 30) of the examination head 66 in the Z direction. With this configuration, the rotation axis 64a and a subject eye E (a circumnutation center) can be made to coincide when viewed from a one-direction side in the Y direction by adjusting X and Z positions of the swing rotation mechanism 64 by the XZ movement mechanism 16. In this case, the swing rotation mechanism 64 rotates (swings) the examination head 66 around the circumnutation center of the subject eye E.

The swing rotation mechanism 64 can also rotate (tilt) the examination head 66 around a rotation axis perpendicular to the Y direction. The rotation is not illustrated.

The examination head 66 is attached to the swing rotation mechanism 64. With this configuration, the examination head 66 is movable in the X, Y, and Z directions by the XZ movement mechanism 16 and the Y movement mechanism 62 and is rotatable in a direction around the rotation axis 64a by the swing rotation mechanism 64. The examination head 66 includes a fundus camera unit 24 and the lens-barrel 28 (including the stereo camera 34) described in the first embodiment.

The control device 40 according to the second embodiment is basically the same as the control device 40 according to the first embodiment except that a method for automatic alignment of the examination head 66 by a drive controlling unit 46 and a method for retraction control of the examination head 66 by a retraction controlling unit 54 are partly different.

The drive controlling unit 46 according to the second embodiment determines an axis TA of tilt based on a tilting angle θ determined by a tilting angle determining unit 43 and then drives the XZ movement mechanism 16, the Y movement mechanism 62, and the swing rotation mechanism 64 to execute the automatic alignment of the examination head 66, like the first embodiment.

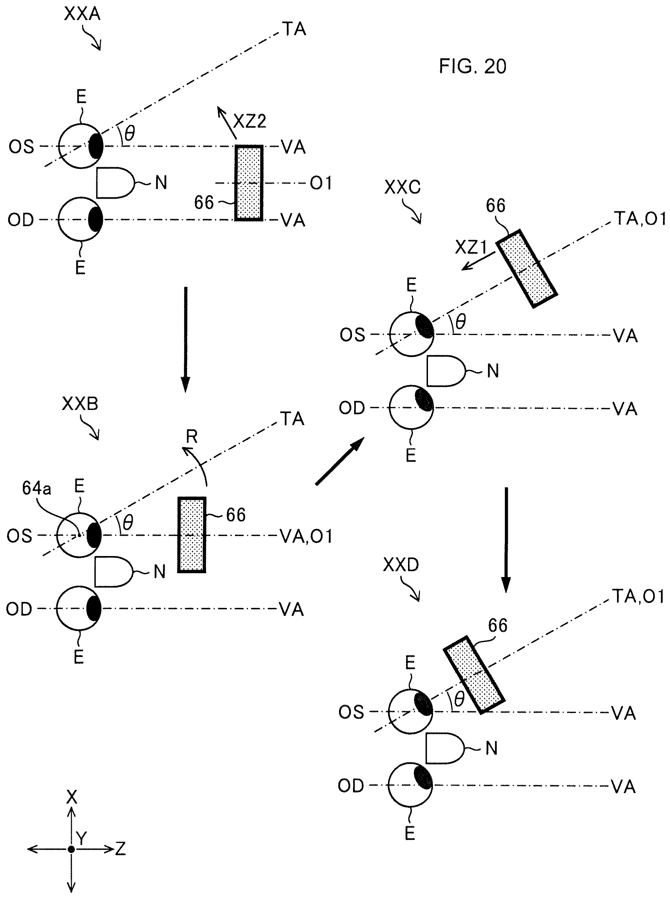

Figure 20 is an explanatory diagram for explaining an example 2-1 of the automatic alignment of the examination head 66 according to the second embodiment. As indicated by reference character XXA in Figure 20, the examination head 66 is arranged at the same poweron initial position P0 as the first embodiment at first.

As indicated by reference characters XXA and XXB in Figure 20, the drive controlling unit 46 drives the XZ movement mechanism 16 to execute a first driving process of moving the examination head 66 (the swing rotation mechanism 64) from the poweron initial position P0 in the X and Z directions (see an arrow XZ2). Specifically, the examination head 66 is moved in the X and Z directions to a position where the rotation axis 64a coincides with the circumnutation center of the subject eye E when viewed from the one-direction side in the Y direction.

The drive controlling unit 46 drives the swing rotation mechanism 64 after completion of the first driving process to execute a second driving process of rotating the examination head 66 around the rotating shaft 20a (the circumnutation center of the subject eye E) by the tilting angle θ (see an arrow R). With this process, as indicated by reference character XXC in Figure 20, the examination head 66 is moved to the axis TA of tilt, and an optical axis O1 becomes parallel to the axis TA of tilt.

The drive controlling unit 46 then drives the XZ movement mechanism 16 after completion of the second driving process to start a third driving process of moving the examination head 66 to an examination position along the axis TA of tilt when the examination head 66 is viewed from the one-direction side in the Y direction (see an arrow XZ1), like the first embodiment. In the third driving process according to the second embodiment, Z-axis movement of the examination head 66 by the XZ movement mechanism 16 is mainly executed, unlike the first embodiment. With this movement, the examination head 66 is moved toward the subject eye E while keeping the tilting angle θ constant.

As indicated by reference character XXD in Figure 20, the drive controlling unit 46 drives the XZ movement mechanism 16 and the Y movement mechanism 62 based on alignment detection performed by an alignment detection unit 44 halfway through the automatic alignment to continue the third driving process until the examination head 66 reaches the examination position.

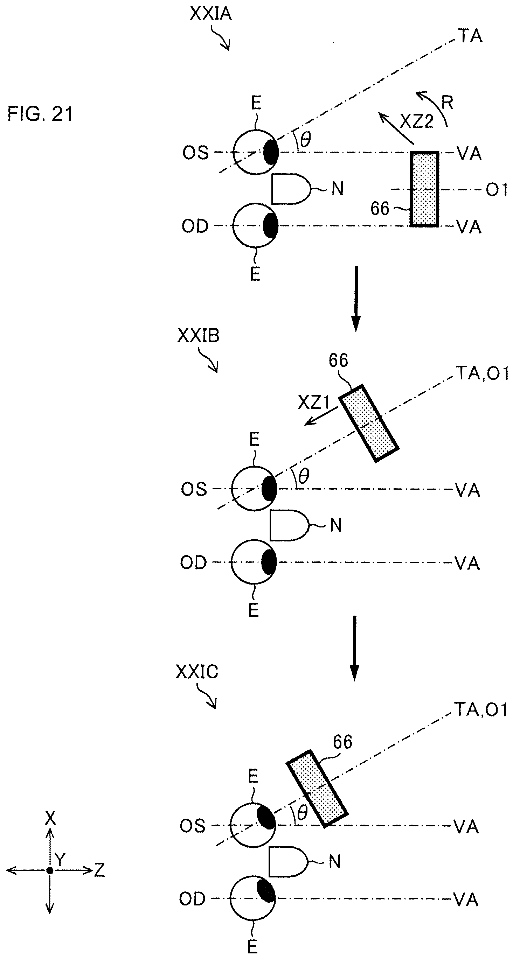

Figure 21 is an explanatory diagram for explaining an example 2-2 of the automatic alignment of the examination head 66 according to the second embodiment. As indicated by reference characters XXIA and XXIB in Figure 21, the drive controlling unit 46 simultaneously drives the XZ movement mechanism 16, the Y movement mechanism 62, and the swing rotation mechanism 64 to execute a first driving process of simultaneously executing movement of the examination head 66 in the X and Z directions and rotation of the examination head 66 by the tilting angle θ (see the arrow XZ2 and the arrow R). The first driving process in the example 2-2 is a process of simultaneously executing the first driving process and the second driving process in the example 2-1 described with reference to Figure 20. With this process, the examination head 66 is moved to the axis TA of tilt, and the optical axis O1 of the objective lens 30 becomes parallel to the axis TA of tilt.

In the first driving process, the examination head 66 may be displaced to a position on the axis TA of tilt where the stereo camera 34 can photograph an anterior eye part of the subject eye E or a position where an observation optical system (not illustrated) inside the examination head 22 can photograph the anterior eye part of the subject eye E via a shortest route.

The drive controlling unit 46 drives the XZ movement mechanism 16 and the Y movement mechanism 62 after completion of the first driving process to execute a second driving process which is the same as the third driving process in the example 2-1, thereby moving the examination head 66 to the examination position along the axis TA of tilt when the examination head 66 is viewed from the one-direction side in the Y direction (see the arrow XZ1). As indicated by reference character XXIC in Figure 21, the drive controlling unit 46 drives the XZ movement mechanism 16 and the Y movement mechanism 62 based on alignment detection performed by the alignment detection unit 44 halfway through the automatic alignment to continue the second driving process until the examination head 66 reaches the examination position.

After completion of examination on a left eye OS (a first eye) by the examination head 66, the retraction controlling unit 54 according to the second embodiment drives the XZ movement mechanism 16 like the first embodiment to retract the examination head 66 along the axis TA of tilt (see Figure 10) or retract the examination head 66 to a rear side in the Z direction or in an outward direction X1 (see Figures 11 and 12). The retraction controlling unit 54 may drive the Y movement mechanism 18 like the first embodiment to retract the examination head 66 toward an upper side in the Y direction (see Figure 14).

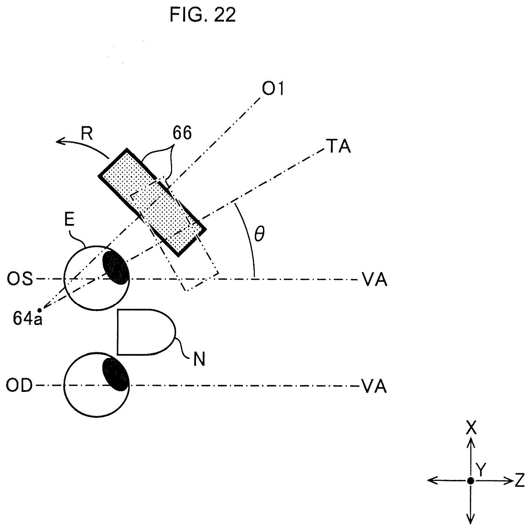

Figure 22 is an explanatory diagram for explaining retraction control to be performed through driving of the swing rotation mechanism 64 by the retraction controlling unit 54 according to the second embodiment. When fundus photographing or OCT imaging of the left eye OS is completed, the retraction controlling unit 54 according to the second embodiment drives the swing rotation mechanism 64 to execute retraction control that rotates the examination head 66 in a direction (see the arrow R) of moving the examination head 66 away from a face (a nose N) of a subject around the rotation axis 64a, as illustrated in Figure 22.

As described above, in the ophthalmic device 10 according to the second embodiment as well, the examination head 66 can be retracted from the face (the nose N) of the subject when examination on one of the left and right subject eyes E is completed. The same effects as those of the above-described first embodiment can be achieved.

### [Third Embodiment]

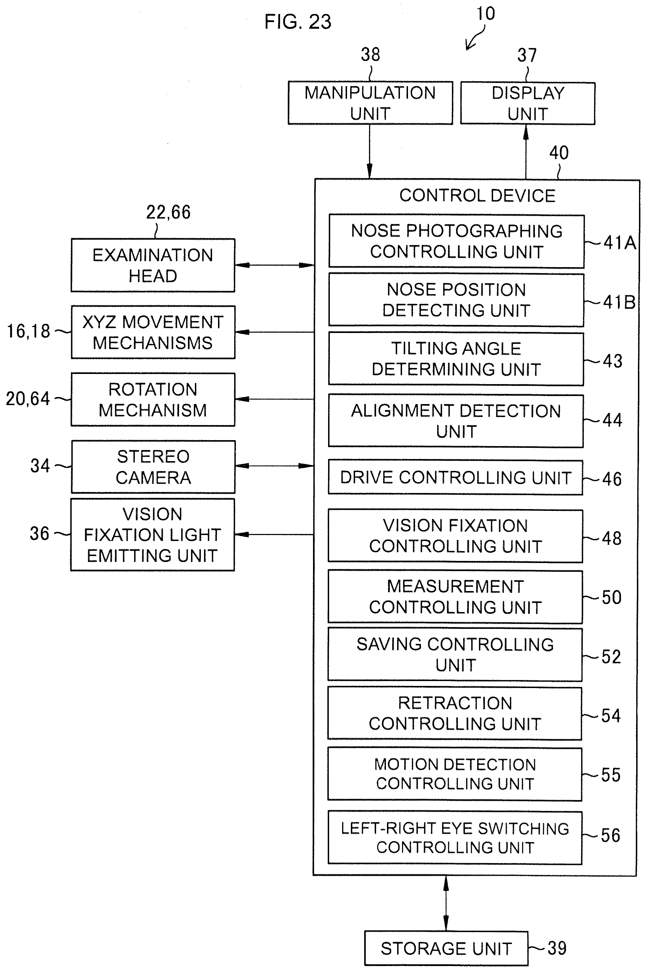

Figure 23 is a block diagram illustrating a configuration of an ophthalmic device 10 according to a third embodiment. In each of the above-described embodiments, the examination head 22 or 66 is retracted after completion of examination on the left eye OS by the examination head 22 or 66, and the examination head 22 or 66 may come closer to the face, the nose N to be specific, of the subject when motion of the face of the subject occurs during the retraction. For this reason, the ophthalmic device 10 according to the third embodiment continuously detects presence or absence of motion of a face of a subject while an examination head 22 or 66 is retracted. When motion of the face occurs during the retraction of the examination head 22 or 66, the ophthalmic device 10 changes a method for retraction control of the examination head 22 or 66.

As illustrated in Figure 23, the ophthalmic device 10 according to the third embodiment is basically the same in configuration as the ophthalmic devices 10 according to the embodiments except that a control device 40 according to the third embodiment functions as a motion detection controlling unit 55 and that a function of a retraction controlling unit 54 is partly different. For this reason, components functionally or structurally identical to those in the ophthalmic devices 10 according to the embodiments are denoted by identical reference numerals, and a description thereof will be omitted.

The motion detection controlling unit 55, together with a stereo camera 34, constitutes a motion detecting unit according to the presently disclosed subject matter. The motion detection controlling unit 55 continuously detects presence or absence of motion of the face of the subject using the stereo camera 34 during retraction of the examination head 22 or 66.

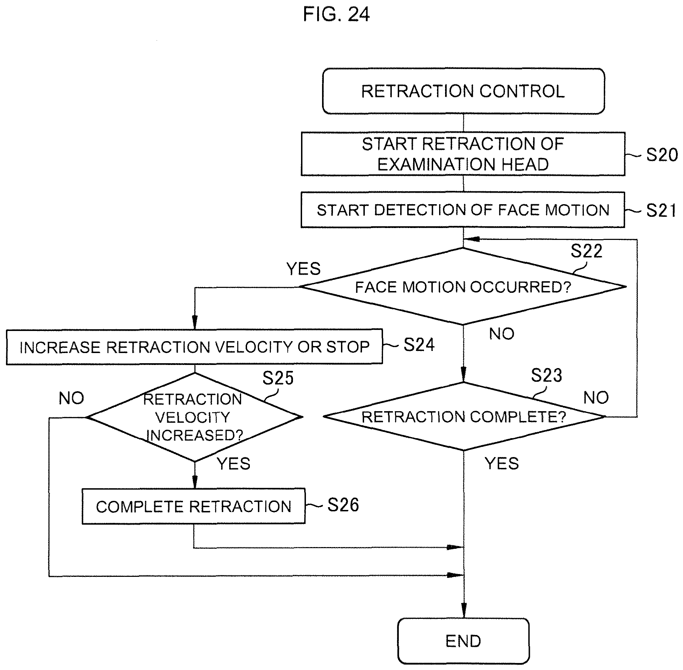

Figure 24 is a flowchart illustrating a flow of the retraction control of the examination head 22 or 66 in the ophthalmic device 10 according to the third embodiment. A case where the retraction controlling unit 54 executes the example 1 of the retraction control illustrated in Figure 10 based on a result of detection by a nose position detecting unit 41B will be described here as an example.

As illustrated in Figure 24, when fundus photographing or OCT imaging of a left eye OS is completed, the retraction controlling unit 54 determines a retraction direction (the one in the example 1 here) based on a result of detection by the nose position detecting unit 41B and then drives an XZ movement mechanism 16 to start retraction of the examination head 22 or 66 from the face (a nose N) of the subject along an axis TA of tilt (step S20).

The motion detection controlling unit 55 continuously executes photographing of the face of the subject by cameras 34a of the stereo camera 34, acquisition of respective photographed images of the face from the cameras 34a, and acquisition of position information (three-dimensional coordinates) of the examination head 22 or 66 at the time of the photographing by the cameras 34a, with the same timing as the start of the retraction of the examination head 22 or 66. Each time the motion detection controlling unit 55 acquires respective photographed images of the face from the cameras 34a and the position information of the examination head 22 or 66, the motion detection controlling unit 55 detects three-dimensional coordinates of a particular part of the face of the subject based on the acquired pieces of data. The motion detection controlling unit 55 detects (determines) presence or absence of motion of the face based on whether a difference (an absolute value) between newly detected three-dimensional coordinates of the particular part and earlier detected three-dimensional coordinates of the particular part is more than a predetermined threshold (step S21).

A method for detecting presence or absence of motion of the face using the stereo camera 34 is not limited to the above-described one and that various publicly known methods may be used.

The detection process by the motion detection controlling unit 55 is repeatedly executed until motion of the face of the subject is detected by the motion detection controlling unit 55 or until the retraction of the examination head 22 or 66 is completed (NO in step S22 and NO in step S23).

When the motion detection controlling unit 55 does not detect motion of the face of the subject before completion of the retraction of the examination head 22 or 66 (NO in step S22 and YES in step S23), the retraction control of the examination head 22 or 66 by the retraction controlling unit 54 is completed. Left-right eye switching control by a left-right eye switching controlling unit 56 described in step S10 of Figure 16 is started.

On the other hand, when the motion detection controlling unit 55 detects motion of the face of the subject before completion of the retraction of the examination head 22 or 66 (YES in step S22), the retraction controlling unit 54 controls the XZ movement mechanism 16 to increase a retraction velocity for the examination head 22 or 66 or stop the retraction of the examination head 22 or 66 (step S24).

When the retraction velocity for the examination head 22 or 66 is increased (YES in step S25), since the examination head 22 or 66 can be retracted from the face, the nose N to be specific, of the subject in a short time, the examination head 22 or 66 is prevented from coming close to the nose N even in the event of motion of the face. When the examination head 22 or 66 is retracted to a predetermined position, the retraction control of the examination head 22 or 66 is completed (step S26).

When the retraction of the examination head 22 or 66 is stopped (NO in step S25), the examination head 22 or 66 is prevented from coming close to the face, the nose N to be specific, of the subject. In this case, the retraction controlling unit 54 switches the retraction control of the examination head 22 or 66 to a manual manipulation mode, and an examiner manipulates a manipulation unit 38 to execute the retraction control of the examination head 22 or 66.

As described above, when motion of the face of the subject occurs during retraction of the examination head 22 or 66, the ophthalmic device 10 according to the third embodiment increases the retraction velocity for the examination head 22 or 66 or stops the retraction. This prevents the examination head 22 or 66 from coming closer to the nose N.

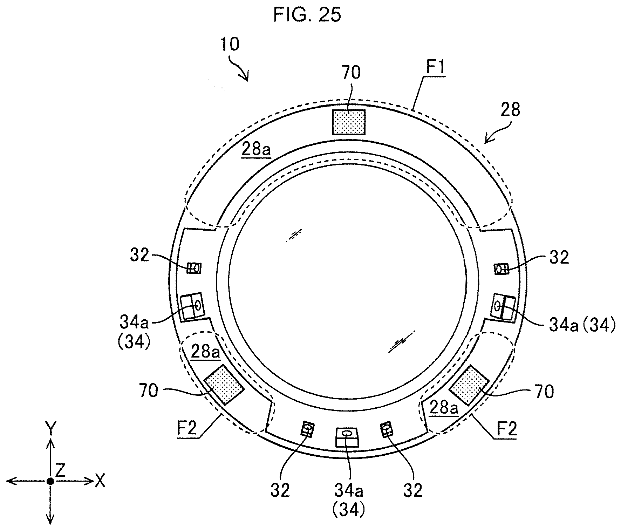

Figure 25 is an explanatory diagram for explaining a modification of a method for detecting presence or absence of motion of the face during retraction of the examination head 22 or 66 in the ophthalmic device 10 according to the third embodiment. Although the motion detection controlling unit 55 according to the third embodiment continuously detects presence or absence of motion of the face of the subject using the stereo camera 34 during retraction of the examination head 22 or 66, one or a plurality of range sensors 70 may be used instead of the stereo camera 34 to continuously detect presence or absence of motion of the face, as illustrated in Figure 25.

The range sensor 70, together with the motion detection controlling unit 55, constitutes a motion detecting unit according to the presently disclosed subject matter. For example, a plurality of range sensors 70 are provided at a lens-barrel distal end face 28a. Each range sensor 70 may be provided at a site other than the lens-barrel distal end face 28a of a lens-barrel 28 or may be provided at a front surface of a housing of the examination head 22 or 66. The number of range sensors 70 may be one. Various publicly known range sensors, such as a photoelectric sensor, an optical fiber sensor, a laser sensor, a camera-equipped laser displacement sensor, an ultrasonic sensor, or a capacitance type sensor, are used as the range sensors 70.

The motion detection controlling unit 55 continuously executes acquisition of detection signals output from the range sensors 70, computation of a face distance which is a distance between the examination head 22 or 66 and the face of the subject based on the detection signals from the range sensors 70, and acquisition of the three-dimensional coordinates of the examination head 22 or 66, during retraction of the examination head 22 or 66. The motion detection controlling unit 55 continuously computes the position information (three-dimensional coordinates) of the face during the retraction of the examination head 22 or 66 based on the three-dimensional coordinates of the examination head 22 or 66 and a result of computation of the face distance during the retraction of the examination head 22 or 66. This allows the motion detection controlling unit 55 to detect (determine) presence or absence of motion of the face based on whether the amount of change in position of the face is more than a predetermined threshold. As a result, the motion detection controlling unit 55 can continuously detect presence or absence of motion of the face of the subject during retraction of the examination head 22 or 66, as with the stereo camera 34.

### [Fourth Embodiment]

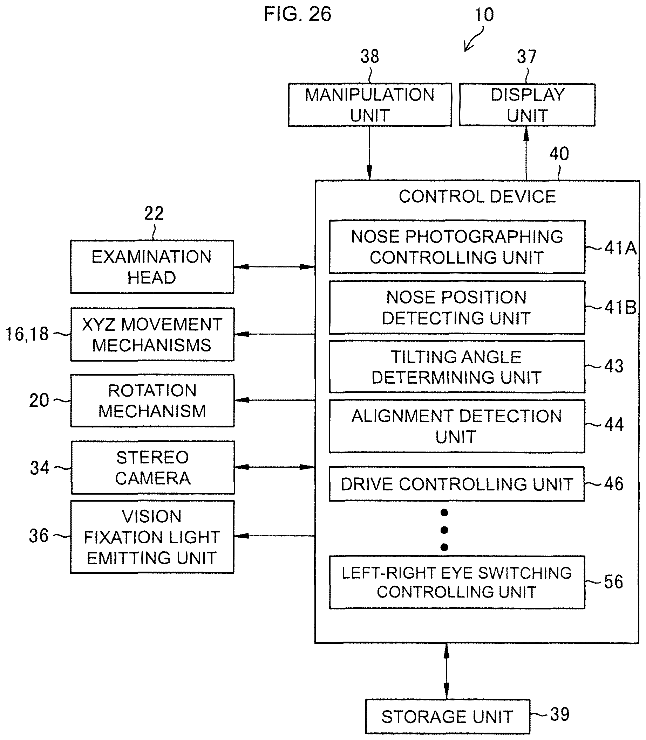

Figure 26 is a block diagram illustrating a configuration of an ophthalmic device 10 according to a fourth embodiment. In the ophthalmic device 10 according to the above-described first embodiment, the examiner selects the tilting angle θ with the manipulation unit 38 before examination on the left eye OS and uses the tilting angle θ at the time of examination on the right eye OD. After completion of the examination on the left eye OS, motion of the face of the subject may occur, i.e., a position of the face may move. When the examination head 22 is displaced to the examination position for the right eye OD along the axis TA of tilt, the examination head 22 may come close to the nose N.

Under the circumstances, the ophthalmic device 10 according to the fourth embodiment detects a position of a nose N of a subject during execution of a left-right movement process by a left-right eye switching controlling unit 56 and determines a tilting angle θ corresponding to a right eye OD (an axis TA of tilt) based on a result of the detection.

As illustrated in Figure 26, the ophthalmic device 10 according to the fourth embodiment is basically the same in configuration as the ophthalmic devices 10 according to the above-described first and third embodiments except that functions of a nose photographing controlling unit 41A, a nose position detecting unit 41B, a tilting angle determining unit 43, and the left-right eye switching controlling unit 56 of a control device 40 are partly different. For this reason, components functionally or structurally identical to those in the ophthalmic devices 10 according to the first and third embodiments are denoted by identical reference numerals, and a description thereof will be omitted.

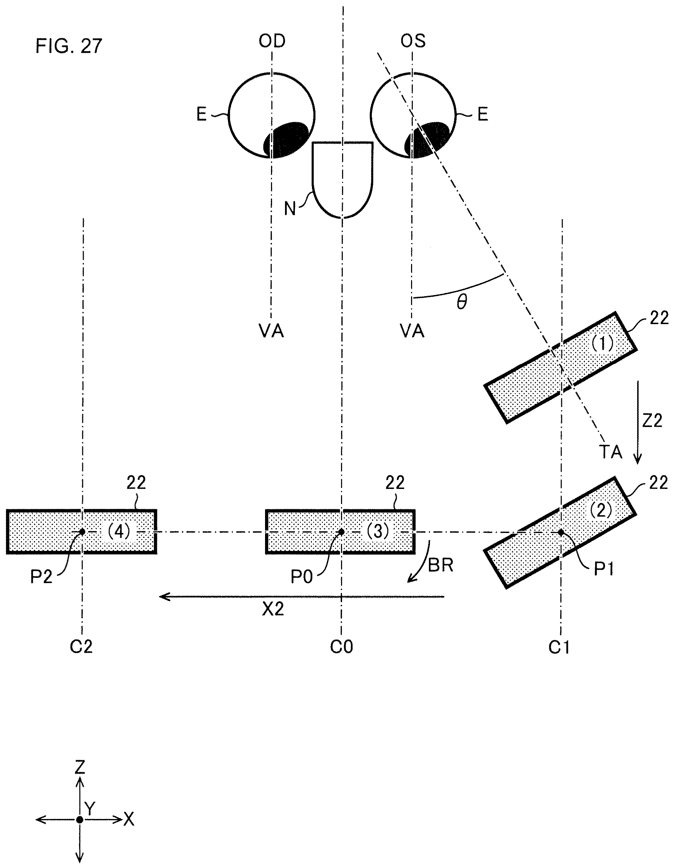

Figure 27 is an explanatory diagram for explaining an example of left-right eye switching control to be executed by the left-right eye switching controlling unit 56 according to the fourth embodiment. As illustrated in Figure 27, the left-right eye switching controlling unit 56 according to the fourth embodiment drives an XZ movement mechanism 16 like the first embodiment to move the examination head 22 after retraction control indicated by the parenthesized number (1) to a pre-examination initial position P1 indicated by the parenthesized number (2) and then execute the left-right movement process. With this process, the examination head 22 is moved from the pre-examination initial position P1 to a pre-examination initial position P2 indicated by the parenthesized number (4) in a left-right switching direction X2.

The left-right eye switching controlling unit 56 drives a swing rotation mechanism 20 halfway through the left-right movement process, as indicated by the parenthesized number (3), to execute a rotation process of rotating the examination head 22 such that an optical axis O1 (see Figure 6, for example) is parallel to a reference axis VA (see an arrow BR). The rotation process is preferably completed before the examination head 22 reaches a centerline C0, i.e., before the examination head 22 reaches a position substantially in front of the nose N.

The nose photographing controlling unit 41A, the nose position detecting unit 41B, and the tilting angle determining unit 43 according to the fourth embodiment perform control related to determination of the tilting angle θ corresponding to the right eye OD after the rotation process of the examination head 22 by the left-right eye switching controlling unit 56.

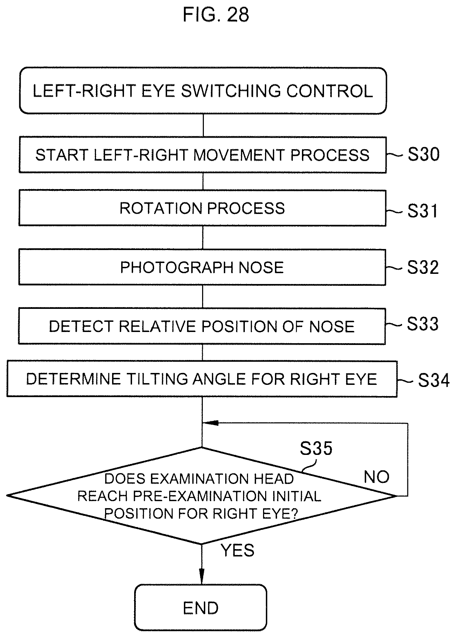

Figure 28 is a flowchart illustrating a flow of a left-right eye switching controlling process by the ophthalmic device 10 according to the fourth embodiment.

As illustrated in Figure 28, the left-right movement process of the examination head 22 by the left-right eye switching controlling unit 56 is started (step S30). When the rotation process of the examination head 22 is executed halfway through the left-right movement process (step S31), the nose photographing controlling unit 41A, the nose position detecting unit 41B, and the tilting angle determining unit 43 of the control device 40 operate.

The nose photographing controlling unit 41A according to the fourth embodiment causes at least two cameras 34a of a stereo camera 34 to execute photographing of the nose N at an arbitrary time after execution of the rotation process by the left-right eye switching controlling unit 56 (step S32). Here, a position at which the stereo camera 34 photographs the nose N is not particularly limited. The stereo camera 34 preferably photographs the nose N near the centerline C0 to ensure photographing of the nose N.

The nose position detecting unit 41B according to the fourth embodiment detects a position (three-dimensional coordinates) of the nose N based on respective photographed images of the nose N photographed by the cameras 34a during the left-right movement process of the examination head 22 and outputs a result of the detection to the tilting angle determining unit 43 (step S33).

The tilting angle determining unit 43 according to the fourth embodiment determines the tilting angle θ that allows avoidance of the examination head 22 coming closer to the nose N based on the result of the detection from the nose position detecting unit 41B (step S34). For example, the tilting angle determining unit 43 computes, for each of a plurality of tilting angles θ different from each other, a nose distance (shortest distance) which is a distance between the examination head 22 and the nose N when the examination head 22 is brought closer to an operating distance determined in advance from the right eye OD. The tilting angle determining unit 43 determines, as the tilting angle θ at the time of examination on the right eye OD, a smallest one among tilting angles θ, for which nose distances are equal to or more than a predetermined threshold, based on a result of the computation of respective nose distances for the tilting angles θ.

The tilting angle determining unit 43 may also determine the tilting angle θ before examination on the left eye OS, based on a result of detection by the nose position detecting unit 41B before the start of automatic alignment described with reference to step S3A in Figure 16.

When the examination head 22 moves to the pre-examination initial position P2, the left-right eye switching controlling unit 56 ends the left-right eye switching control (step S35). With the end, automatic alignment of the examination head 22 with the right eye OD is started under control by the drive controlling unit 46, like the first embodiment. A drive controlling unit 46 according to the fourth embodiment determines the axis TA of tilt corresponding to the right eye OD based on the tilting angle θ for the right eye OD determined by the tilting angle determining unit 43 and displaces the examination head 22 to an examination position for the right eye OD along the axis TA of tilt.

As described above, the ophthalmic device 10 according to the fourth embodiment can determine the tilting angle θ (the axis TA of tilt) corresponding to a position of a face (the nose N) of the subject before examination on the right eye OD based on a detection result of detecting the position of the subject's nose N during the left-right movement process after examination on the left eye OS. As a result, even if motion of the face of the subject occurs after completion of the examination on the left eye OS, the examination head 22 during the automatic alignment with the right eye OD is prevented from coming closer to the nose N.

### [Fifth Embodiment]

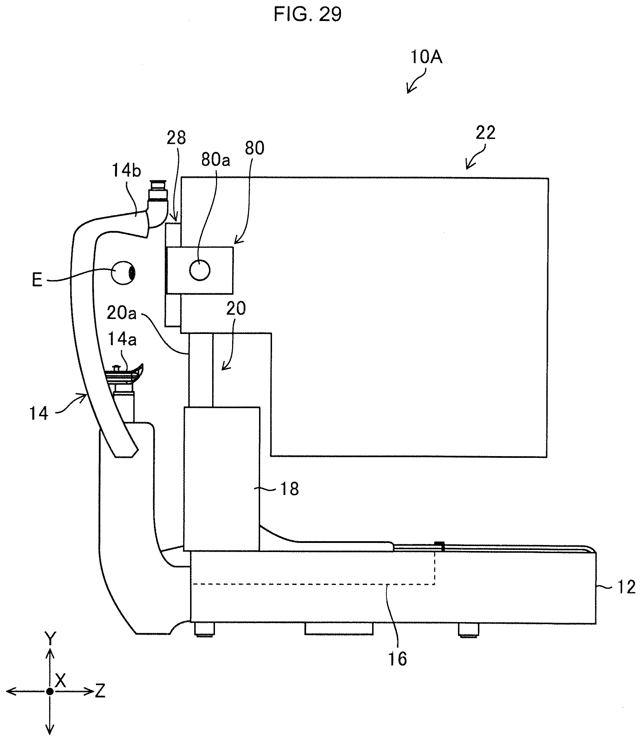

Figure 29 is a side view of an ophthalmic device 10A according to a fifth embodiment. The ophthalmic device 10 (see Figure 1) according to the above-described first embodiment includes the swing rotation mechanism 20 having the rotating shaft 20a parallel to the Y direction, and brings the examination head 22 closer to the subject eye E along the axis TA of tilt obtained by tilting the reference axis VA in the X direction (the outward direction X1) around the subject eye E at the time of automatic alignment of the examination head 22. In contrast, the ophthalmic device 10A according to the fifth embodiment brings an examination head 22 closer to a subject eye E along an axis TA of tilt obtained by tilting a reference axis VA in a direction other than an X direction around the subject eye E at the time of automatic alignment of the examination head 22.

As illustrated in Figure 29, the ophthalmic device 10A according to the fifth embodiment is basically the same in configuration as the ophthalmic devices 10 according to the first, third, and fourth embodiments except that the ophthalmic device 10A includes a tilt rotation mechanism 80 and executes the automatic alignment of the examination head 22 differently from the first embodiment. For this reason, components functionally or structurally identical to those in the ophthalmic devices 10 according to the embodiments are denoted by identical reference numerals, and a description thereof will be omitted.

The tilt rotation mechanism 80 corresponds to a rotation mechanism according to the presently disclosed subject matter and, together with an XZ movement mechanism 16, a Y movement mechanism 18, and a swing rotation mechanism 20, constitutes a displacement mechanism according to the presently disclosed subject matter. The tilt rotation mechanism 80 includes a rotating shaft 80a parallel to a Y direction and an electric drive mechanism which rotates the rotating shaft 80a, and rotates (tilts) the examination head 22 around the rotating shaft 80a.

A position of the rotating shaft 80a and a position of an objective lens 30 coincide (the term "coincide" as used herein is intended to include the meaning of "coincide substantially"; the same applies hereinafter) when the rotating shaft 80a is viewed from a one-direction side in an axial direction of the rotating shaft 80a. With this configuration, the examination head 22 is rotated (tilted) around the objective lens 30 by the tilt rotation mechanism 80.

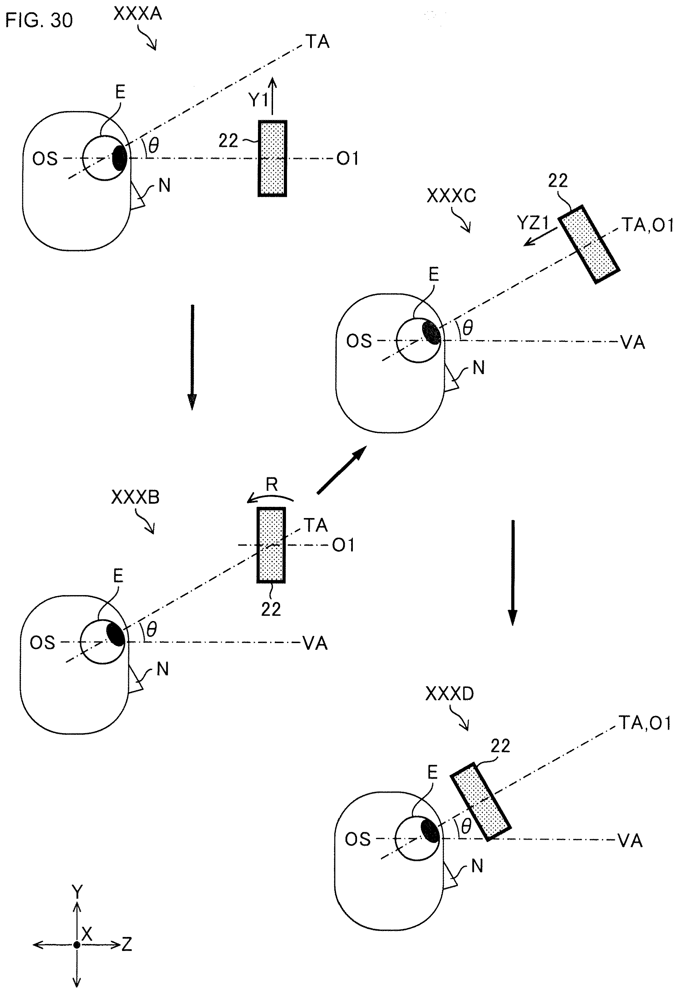

As described above, the examination head 22 according to the fifth embodiment is biaxially rotatable (swingable and tiltable) around the objective lens 30 by the swing rotation mechanism 20 and the tilt rotation mechanism 80. The examination head 22 according to the fifth embodiment is thus rotatable around an arbitrary rotating shaft (including ones other than a rotating shaft 20a and the rotating shaft 80a) perpendicular to a Z direction by driving at least one of the swing rotation mechanism 20 and the tilt rotation mechanism 80. For this reason, in the fifth embodiment, a direction (which is a direction perpendicular to the Z direction and away from a nose N) other than the outward direction X1 according to the first embodiment, such as an upward direction of the Y direction, is set as an outward direction Y1 (see Figure 30), and an axis obtained by tilting the reference axis VA in the outward direction Y1 around the subject eye E is set as the axis TA of tilt.

A control device 40 according to the fifth embodiment is basically the same as the control device 40 according to the first embodiment except that a direction of tilt of the axis TA of tilt is different from that in the first embodiment.

A tilting angle determining unit 43 according to the fifth embodiment determines a tilting angle θ in the outward direction Y1 (see Figure 30) of the axis TA of tilt with respect to the reference axis VA when viewed from a one-direction side in the X direction, i.e., the tilting angle θ of the axis TA of tilt with respect to the reference axis VA in a YZ plane. A specific method for determining the tilting angle θ is the same as the method for determining the tilting angle θ according to the first embodiment except that the direction of tilt of the axis TA of tilt is different and that a specific description thereof will be omitted.

A drive controlling unit 46 according to the fifth embodiment determines the axis TA of tilt based on the tilting angle θ determined by the tilting angle determining unit 43 and then drives the XZ movement mechanism 16, the Y movement mechanism 18, the swing rotation mechanism 20, and the tilt rotation mechanism 80 to execute the automatic alignment of the examination head 22.

Figure 30 is an explanatory diagram for explaining an example 3 of the automatic alignment of the examination head 22 according to the fifth embodiment. The example 3 is basically the same as the example 1-1 (see Figure 6) described in the first embodiment except that the direction of tilt of the axis TA of tilt is different.

As indicated by reference character XXXA in Figure 30, the examination head 22 is arranged at the same initial position as the first embodiment at first. The drive controlling unit 46 according to the fifth embodiment drives the XZ movement mechanism 16 and the Y movement mechanism 18 to execute a first driving process of moving the examination head 22 from the initial position to the axis TA of tilt in the outward direction Y1 (the upward direction of the Y direction) when the examination head 22 is viewed from the one-direction side in the X direction.

As indicated by reference character XXXB in Figure 30, the drive controlling unit 46 drives the tilt rotation mechanism 80 after completion of the first driving process to execute a second driving process of rotating the examination head 22 around the rotating shaft 80a by the tilting angle θ (see an arrow R). With this process, as indicated by reference character XXXC in Figure 30, an optical axis O1 of the examination head 22 (the objective lens 30) becomes parallel to the axis TA of tilt. The second driving process may be executed before the first driving process.

The drive controlling unit 46 then drives the XZ movement mechanism 16 and the Y movement mechanism 18 after completion of the second driving process to start a third driving process of moving the examination head 22 to an examination position along the axis TA of tilt when the examination head 22 is viewed from the one-direction side in the X direction (see an arrow YZ1). With this process, the examination head 22 is moved toward the subject eye E while keeping the tilting angle θ constant (the term "constant" as used herein is intended to include the meaning of "substantially constant"; the same applies hereinafter).

As indicated by reference character XXXD in Figure 30, the drive controlling unit 46 drives the XZ movement mechanism 16 and the Y movement mechanism 18 based on alignment detection performed by an alignment detection unit 44 halfway through the automatic alignment to continue the third driving process until the examination head 22 reaches the examination position.

The drive controlling unit 46 switches an alignment mode of the examination head 22 to a manual alignment mode when the alignment detection unit 44 is incapable of alignment detection during a period from the start of the automatic alignment or the start of the third driving process to movement of the examination head 22 for a fixed time determined in advance or over a fixed distance.

Like the example 1-2 (see Figure 7) of the automatic alignment of the examination head 22 according to the first embodiment, the first driving process may be started after the examination head 22 is first moved toward a front side in the Z direction (a subject eye E side) by a predetermined distance. Like the example 1-3 (see Figure 8) of the automatic alignment of the examination head 22 according to the first embodiment, a first driving process of simultaneously executing movement of the examination head 22 toward the front side in the Z direction and in the outward direction Y1 and rotation of the examination head 22 by the tilting angle θ may be executed.

Processes (e.g., examination head retraction control by the retraction controlling unit 54 and left-right eye switching control by the left-right eye switching controlling unit 56) after the automatic alignment of the examination head 22 are basically the same as those in the embodiments, and a specific description thereof will be omitted.

As described above, in the ophthalmic device 10 according to the fifth embodiment as well, the examination head 22 can be moved to the examination position for the subject eye E from an oblique direction (obliquely upward direction) along the axis TA of tilt at the time of the automatic alignment. As a result, the same effects as those of the embodiments can be achieved.

The ophthalmic device 10 brings the examination head 22 closer to the subject eye E along the axis TA of tilt obtained by tilting the reference axis VA in the outward direction Y1 (the upward direction of the Y direction) around the subject eye E at the time of the automatic alignment of the examination head 22 in the fifth embodiment. The direction of tilt of the axis TA of tilt is not particularly limited as long as the direction is a direction perpendicular to the Z direction and away from the nose N.

When the direction of tilt of the axis TA of tilt is fixed to the outward direction Y1 in the ophthalmic device 10A according to the fifth embodiment, the swing rotation mechanism 20 may be omitted.

### [Sixth Embodiment]

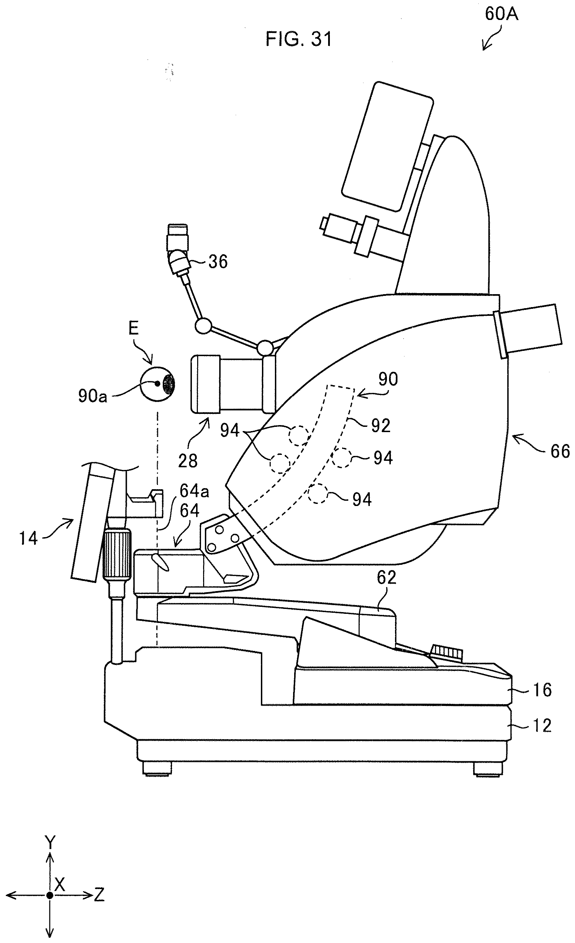

Figure 31 is a side view of an ophthalmic device 60A according to a sixth embodiment. The ophthalmic device 10 (see Figure 19) according to the above-described second embodiment includes the swing rotation mechanism 64 having the rotation axis 64a parallel to the Y direction, and brings the examination head 66 closer to the subject eye E along the axis TA of tilt obtained by tilting the reference axis VA in the X direction (the outward direction X1) around the subject eye E at the time of the automatic alignment of the examination head 66. In contrast, the ophthalmic device 60A according to the sixth embodiment brings an examination head 66 closer to a subject eye E along an axis TA of tilt obtained by tilting a reference axis VA in a direction other than an X direction around the subject eye E at the time of automatic alignment of the examination head 66, like the ophthalmic device 10A according to the above-described fifth embodiment.

As illustrated in Figure 31, the ophthalmic device 60A according to the sixth embodiment is basically the same in configuration as the ophthalmic devices 60 according to the second and third embodiments except that the ophthalmic device 60A includes a tilt rotation mechanism 90 and executes the automatic alignment of the examination head 66 differently from the second embodiment. For this reason, components functionally or structurally identical to those in the ophthalmic devices 60 according to the embodiments are denoted by identical reference numerals, and a description thereof will be omitted.

The tilt rotation mechanism 90 corresponds to a rotation mechanism according to the presently disclosed subject matter and, together with an XZ movement mechanism 16, a Y movement mechanism 62, and a swing rotation mechanism 64, constitutes a displacement mechanism according to the presently disclosed subject matter. The tilt rotation mechanism 90 rotates (tilts) the examination head 66 around an imaginary rotation axis 90a perpendicular to a Y direction.

The tilt rotation mechanism 90 includes, for example, a curved arm 92, a plurality of guide wheel portions 93, and a head moving mechanism (not illustrated). The curved arm 92 is fixed to the swing rotation mechanism 64 and has the shape of an arc of a circle centered at the rotation axis 90a.

The plurality of guide wheel portions 93 are held inside the examination head 66 so as to be rotatable around center axes parallel to the rotation axis 90a. The guide wheel portions 93 are arranged such that the guide curved arm 92 is sandwiched therebetween in the Y direction. With this configuration, the examination head 66 is movable along the curved arm 92.

The head moving mechanism (not illustrated) of the tilt rotation mechanism 90 is provided inside the examination head 66 and moves the examination head 66 along the curved arm 92. A configuration of the head moving mechanism is a publicly known technique (e.g., Japanese Patent Application Laid-Open No. 2022-112637) and that a specific description thereof will be omitted. The movement of the examination head 66 along the curved arm 92 by the head moving mechanism allows rotation (tilting) of the examination head 66 around the rotation axis 90a. Additionally, alignment of the rotation axis 90a with the subject eye E allows rotation (tilting) of the examination head 66 around the subject eye E.

As described above, the examination head 66 according to the sixth embodiment is biaxially rotatable (swingable and tiltable) by the swing rotation mechanism 64 and the tilt rotation mechanism 90, like the examination head 22 according to the fifth embodiment. The examination head 66 according to the sixth embodiment is thus rotatable around an arbitrary rotation axis (including ones other than a rotation axis 64a and the rotation axis 90a) perpendicular to a Z direction by driving at least one of the swing rotation mechanism 64 and the tilt rotation mechanism 90. For this reason, in the sixth embodiment, a direction (which is a direction perpendicular to the Z direction and away from a nose N) other than the outward direction X1 according to the second embodiment, such as an upward direction of the Y direction, is set as an outward direction Y1, and an axis obtained by tilting the reference axis VA in the outward direction Y1 around the subject eye E is set as the axis TA of tilt, like the fifth embodiment.

A control device 40 according to the sixth embodiment is basically the same as the control device 40 according to the second embodiment except that a direction of tilt of the axis TA of tilt is different from that in the second embodiment.

A tilting angle determining unit 43 according to the sixth embodiment determines a tilting angle θ in the outward direction Y1 of the axis TA of tilt with respect to the reference axis VA, like the tilting angle determining unit 43 according to the fifth embodiment.

A drive controlling unit 46 according to the sixth embodiment determines the axis TA of tilt based on the tilting angle θ determined by the tilting angle determining unit 43 and then drives the XZ movement mechanism 16, the Y movement mechanism 62, the swing rotation mechanism 64, and the tilt rotation mechanism 90 to execute the automatic alignment of the examination head 66.

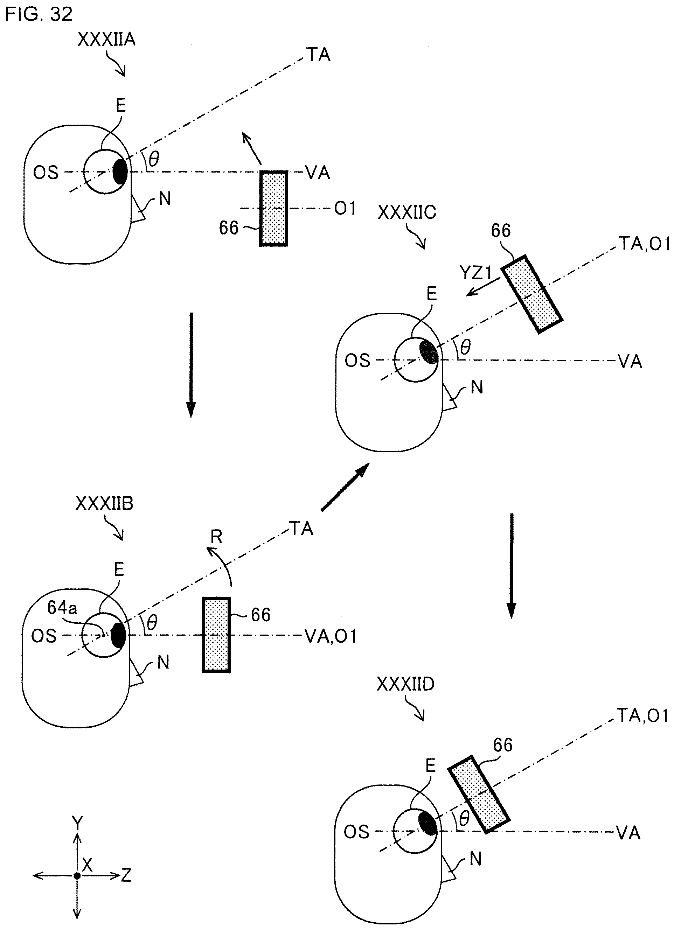

Figure 32 is an explanatory diagram for explaining an example 4 of the automatic alignment of the examination head 66 according to the sixth embodiment. The example 4 is basically the same as the example 2-1 (see Figure 20) described in the second embodiment except that the direction of tilt of the axis TA of tilt is different. As indicated by reference character XXXIIA in Figure 32, the examination head 66 is arranged at the same initial position as the first embodiment at first.

As indicated by reference characters XXXIIA and XXXIIB in Figure 32, the drive controlling unit 46 drives the XZ movement mechanism 16 and the Y movement mechanism 62 to execute a first driving process of moving, in the X, Y, and Z directions, the examination head 66 to a position where the rotation axis 64a and the rotation axis 90a coincide with a circumnutation center of the subject eye E.

The drive controlling unit 46 drives the tilt rotation mechanism 90 after completion of the first driving process to execute a second driving process of rotating the examination head 66 in the outward direction Y1 around the rotation axis 90a (the circumnutation center of the subject eye E) by the tilting angle θ (see an arrow R). With this process, as indicated by reference character XXXIIC in Figure 32, the examination head 66 is moved to the axis TA of tilt, and an optical axis O1 becomes parallel to the axis TA of tilt.

The drive controlling unit 46 then drives the XZ movement mechanism 16 and the Y movement mechanism 62 after completion of the second driving process to start a third driving process of moving the examination head 66 to an examination position along the axis TA of tilt when the examination head 66 is viewed from a one-direction side in the X direction (see an arrow YZ1). With this process, the examination head 66 is moved toward the subject eye E while keeping the tilting angle θ constant (the term "constant" as used herein is intended to include the meaning of "substantially constant"; the same applies hereinafter).

As indicated by reference character XXXIID in Figure 32, the drive controlling unit 46 drives the XZ movement mechanism 16 and the Y movement mechanism 62 based on alignment detection performed by an alignment detection unit 44 halfway through the automatic alignment to continue the third driving process until the examination head 66 reaches the examination position.

The drive controlling unit 46 switches an alignment mode of the examination head 66 to a manual alignment mode when an alignment detection unit 44 is incapable of alignment detection during a period from the start of the automatic alignment or the start of the third driving process to movement of the examination head 66 for a fixed time determined in advance or over a fixed distance.

The first driving process and the second driving process may be simultaneously executed, like the example 2-2 (see Figure 21) of the automatic alignment of the examination head 66 according to the second embodiment.

Processes (e.g., examination head retraction control by a retraction controlling unit 54 and left-right eye switching control by a left-right eye switching controlling unit 56) after the automatic alignment of the examination head 66 are basically the same as those in the embodiments, and a specific description thereof will be omitted.

As described above, in the ophthalmic device 10 according to the sixth embodiment as well, the examination head 66 can be moved to the examination position for the subject eye E from an oblique direction (obliquely upward direction) along the axis TA of tilt at the time of the automatic alignment. As a result, the same effects as those of the embodiments can be achieved.

The ophthalmic device 10 brings the examination head 66 closer to the subject eye E along the axis TA of tilt obtained by tilting the reference axis VA in the outward direction Y1 (the upward direction of the Y direction) around the subject eye E at the time of the automatic alignment of the examination head 66 in the sixth embodiment. The direction of tilt of the axis TA of tilt around the subject eye E is not particularly limited as long as the direction is a direction perpendicular to the Z direction and away from the nose N.

When the direction of tilt of the axis TA of tilt is fixed to the outward direction Y1 in the ophthalmic device 10A according to the sixth embodiment, the swing rotation mechanism 64 may be omitted.

### [Others]

Although the examination head 22 or 66 is moved to the examination position for the subject eye E along the axis TA of tilt with the tilting angle θ determined by the tilting angle determining unit 43 at the time of automatic alignment in each of the above-described embodiments, the examination head 22 or 66 may be moved to the examination position for the subject eye E along the axis TA of tilt with the tilting angle θ at the time of manual alignment. Even in this case, retraction control by the retraction controlling unit 54 is automatically executed after examination on the left eye OS (the first eye).

Although examination on the right eye OD is executed after examination on the left eye OS is first executed in each embodiment, the examination on the left eye OS may be executed after the examination on the right eye OD is first executed.

Although a case where a displacement mechanism which displaces the examination head 22 or 66 with respect to the subject eye E includes the XZ movement mechanism 16, the Y movement mechanism 18 or 62, and the swing rotation mechanism 20 or 64 and a case where the displacement mechanism includes the tilt rotation mechanism 80 or 90 in addition to the listed components have been described as examples in the embodiments, a configuration and the type of the displacement mechanism are not particularly limited. For example, a robot arm (multijoint arm) may be used as a displacement mechanism according to the presently disclosed subject matter.

Although a multifunction machine which is a combination of a fundus camera and an optical coherence tomograph and a fundus camera alone have been described as examples of the ophthalmic device 10 in the embodiments, the presently disclosed subject matter is not limited to this. The presently disclosed subject matter can also be applied to various ophthalmic devices (including devices which perform various procedures on the subject eye E, such as a laser surgery device) which are used for examination (ocular characteristics measurement, photographing, and observation) on the left and right subject eyes E, such as an optical coherence tomograph alone and an SLO device.

### {Reference Signs List}

10 ophthalmic device
12 base
14 face support
14a chin rest
14b forehead rest
16 XZ movement mechanism
18 Y movement mechanism
20 swing rotation mechanism
20a rotating shaft
22 examination head
24 fundus camera unit
26 OCT unit
28 lens-barrel
28a lens-barrel distal end face
30 objective lens
32 illumination light source
34 stereo camera
34a camera
36 vision fixation light emitting unit
37 display unit
38 manipulation unit
39 storage unit
40 control device
41A nose photographing controlling unit
41B nose position detecting unit
43 tilting angle determining unit
44 alignment detection unit
46 drive controlling unit
47A detection controlling unit
47B retraction controlling unit
48 vision fixation controlling unit
50 measurement controlling unit
51A retraction controlling unit
51B left-right eye switching controlling unit
52 saving controlling unit
54 retraction controlling unit
55 motion detection controlling unit
56 left-right eye switching controlling unit
60 ophthalmic device
62 Y movement mechanism
64 swing rotation mechanism
64a rotation axis
66 examination head
70 range sensor
80, 90 tilt rotation mechanism
80a, 90a rotating shaft, rotation axis
100 objective lens
102 objective lens
104 examination head
C0 centerline
E subject eye
F1 upper region
F2 lower region
H subject
N nose
O1 optical axis
OD right eye
OS left eye
P0 poweron initial position
P1 pre-examination initial position
P2 pre-examination initial position
TA axis of tilt
VA reference axis
X1 outward direction
X2 left-right switching direction
XZ1 arrow
XZ2 arrow
XZ3 arrow
Y1 outward direction
Z1 arrow
Z2 rearward direction
d1 operating distance
d2 operating distance
θ tilting angle

## Claims

1. An ophthalmic device comprising:
an examination head configured to examine subject eyes, the ophthalmic device configured to use the examination head to examine a first eye that is one of left and right ones of the subject eyes and then examine a second eye that is the other of the left and right subject eyes;
a displacement mechanism configured to displace the examination head with respect to the subject eyes;
a drive controlling unit configured to drive the displacement mechanism to displace the examination head to an examination position for the subject eye under examination along an axis of tilt, wherein the axis of tilt is an axis obtained by tilting, around a center of one subject eye under examination, a reference axis that extends along an eye direction of the subject eye in parallel with a front-back direction serving as an operating distance direction of the examination head, outward away from a subject's nose; and
a retraction controlling unit configured to, when examination on the first eye by the examination head is completed, drive the displacement mechanism to retract the examination head in a direction away from a face of the subject.

2. The ophthalmic device according to claim 1, wherein the retraction controlling unit drives the displacement mechanism to retract the examination head in a direction away from the nose in the face.

3. The ophthalmic device according to claim 1, wherein the retraction controlling unit drives the displacement mechanism to retract the examination head in a direction away from the face along the axis of tilt.

4. The ophthalmic device according to claim 1, wherein
the displacement mechanism includes
a movement mechanism configured to move the examination head in the front-back direction, a left-right direction, and an up-down direction with respect to the subject eyes, and
a rotation mechanism configured to rotate the examination head around a rotation axis determined in advance, and
when a direction, in which the examination head moves away from the face, of the front-back direction is a rearward direction, the retraction controlling unit drives the movement mechanism to move the examination head in at least one of the rearward direction and the outward direction.

5. The ophthalmic device according to claim 2, wherein
the displacement mechanism includes
a movement mechanism configured to move the examination head in the front-back direction, a left-right direction, and an up-down direction with respect to the subject eyes,
and
a rotation mechanism configured to rotate the examination head around a rotation axis determined in advance, and
the retraction controlling unit drives the rotation mechanism to rotate the examination head in a direction away from the nose.

6. The ophthalmic device according to claim 2, wherein
the retraction controlling unit drives the displacement mechanism to retract the examination head in an upward direction.

7. The ophthalmic device according to any one of claims 1 to 6, comprising:
a photographing unit configured to photograph the subject's nose, and
a nose position detecting unit configured to detect a position of the nose based on a photographed image of the nose photographed by the photographing unit, wherein
the retraction controlling unit drives the displacement mechanism based on a result of detection from the nose position detecting unit to retract the examination head in a direction away from the face of the subject.

8. The ophthalmic device according to any one of claims 1 to 6, comprising
a motion detecting unit configured to continuously detect presence or absence of motion of the face while the retraction controlling unit drives the displacement mechanism to retract the examination head in a direction away from the face, wherein
the retraction controlling unit stops driving of the displacement mechanism or increases a velocity of retraction of the examination head by the displacement mechanism when motion of the face is detected by the motion detecting unit.

9. The ophthalmic device according to claim 4 or 5, wherein
the rotation axis is parallel to the up-down direction, and
the outward direction is parallel to the left-right direction.

10. The ophthalmic device according to claim 4 or 5, wherein
the rotation axis is perpendicular to the up-down direction, and
the outward direction is an upward direction of the up-down direction.

11. The ophthalmic device according to any one of claims 1 to 6, wherein
the displacement mechanism includes
a movement mechanism configured to move the examination head in the front-back direction, a left-right direction, and an up-down direction with respect to the subject eyes, and
a rotation mechanism configured to rotate the examination head around a rotation axis determined in advance,
the examination head includes an objective lens,
the drive controlling unit executes
a process of driving the movement mechanism to move the examination head from respective initial positions determined in advance for the left and right subject eyes to the axis of tilt,
a process of driving the rotation mechanism to rotate the examination head and make an optical axis of the objective lens parallel to the axis of tilt, and
a process of driving the movement mechanism to displace the examination head to the examination position along the axis of tilt, and
the device comprises a left-right eye switching controlling unit configured to, after the retraction controlling unit drives the displacement mechanism to retract the examination head in a direction away from the face, drive the displacement mechanism to displace the examination head to a second initial position that is the initial position for the second eye via a first initial position that is the initial position for the first eye or to displace the examination head to the second initial position not via the first initial position.

12. The ophthalmic device according to claim 11, wherein
the rotation mechanism has the rotation axis parallel to the up-down direction and rotates the examination head around the objective lens,
the first initial position and the second initial position are spaced in the left-right direction when viewed from a one-direction side in the up-down direction,
the left-right eye switching controlling unit executes, in displacing the examination head from the first initial position to the second initial position, a left-right movement process of driving the movement mechanism to move the examination head at least in the left-right direction and a rotation process of making the optical axis parallel to the reference axes by driving the rotation mechanism to rotate the examination head during the left-right movement process, and
the device comprises
a photographing unit provided at the examination head and configured to photograph the subject's nose after execution of the rotation process,
a nose position detecting unit configured to detect a position of the nose based on a photographed image of the nose photographed by the photographing unit, and
a tilting angle determining unit configured to determine a tilting angle of the axis of tilt with respect to the reference axis corresponding to the second eye based on a result of detection from the nose position detecting unit.

13. A method for operating an ophthalmic device comprising
an examination head configured to examine subject eyes, and
a displacement mechanism configured to displace the examination head with respect to the subject eyes,
wherein the ophthalmic device is configured to use the examination head to examine a first eye that is one of left and right ones of the subject eyes and then examine a second eye that is the other of the left and right subject eyes, the method comprising:
a drive controlling step of driving the displacement mechanism to displace the examination head to an examination position for the subject eye under examination along an axis of tilt, wherein the axis of tilt is an axis obtained by tilting, around a center of one subject eye under examination, a reference axis that extends along an eye direction of the subject eye in parallel with a front-back direction serving as an operating distance direction of the examination head, outward away from a subject's nose; and
a retraction controlling step of, when examination on the first eye by the examination head is completed, driving the displacement mechanism to retract the examination head in a direction away from a face of the subject.
